# EUROPEAN PATENT APPLICATION

(11) **EP 4 696 494 A1**
(43) Date of publication of application: **18.02.2026**
(21) Application number: 24780089.9
(22) Date of filing: 23.03.2024
(51) Int. Cl.: B32B 5/18, B32B 27/00, B32B 9/00

(54) **LAMINATE COMPRISING TWO-DIMENSIONAL LAYERED SUBSTANCE AND POROUS FILM, METHOD FOR TRANSFERRING LAMINATE, AND METHOD FOR MANUFACTURING LAMINATE**

(30) Priority: 24.03.2023 JP 2023047649
(71) Applicant: Watanabe, Takeshi, Sagamihara-shi, Kanagawa 252-5258 (JP)
(72) Inventor: KOH Shinji, Sagamihara-shi, Kanagawa 252-5258 (JP); WATANABE Takeshi, Sagamihara-shi, Kanagawa 252-5258 (JP)
(74) Representative: Wasner, Marita
(86) International application number: PCT/JP2024/011525
(87) International publication number: WO 2024/203971

(57) **Abstract**

[Problem] The present invention addresses the problem of providing a method whereby it is possible to easily transfer a high-quality two-dimensional layered substance to a desired substrate without requiring floating on the surface of water and without causing damage during the transfer process; and a laminate that is used in the method.

[Solution] The present invention provides a laminate comprising a two-dimensional layered substance and a porous film laminated on the two-dimensional layered substance, the laminate being capable of self-support while maintaining a planar structure in the atmosphere.

## Description

### Technical Field

The present invention relates to a laminated body comprising a graphene film laminated with a porous membrane having micropores, a method for transferring the laminated body, and a method for producing the laminated body.

### Background Art

Graphene has excellent properties such as optical transparency and high electrical conductivity, and is therefore expected to be applied to transparent conductive films and the like. Various methods have been developed for producing graphene; among them, chemical vapor deposition (CVD), which enables large-area fabrication of high-quality graphene films, is particularly useful for applications. In CVD growth of graphene, gases such as methane serving as the carbon source are used to form a graphene film on a metal catalyst substrate such as copper. To apply the resulting graphene to transparent conductive films or various devices, it is necessary to transfer the graphene from the metal catalyst substrate onto a desired substrate. Graphene is a sheet material with the thickness of a single atomic layer and cannot be self-supporting. Accordingly, to transfer graphene, either (i) a method is employed in which graphene present on a catalytic metal is attached to a desired substrate, or (ii) a method is employed in which a transfer support film such as a polymer is first applied on graphene. In either method, a process of separating or dissolving the metal catalyst substrate is required. The necessity of this transfer process is one factor hindering the industrial applications of graphene. In addition to process complexity, there are issues such as damage to graphene (e.g. tearing) and residue of the transfer polymer.

In typical transfer of CVD graphene, polymethyl methacrylate (PMMA) is used as a transfer support film. In this method, a PMMA film is formed on the graphene on a metal catalyst (copper) by spin-coating a PMMA solution or the like. Then, the laminated body of PMMA, graphene, and copper is floated on a copper etchant, with the copper side in contact with the etchant, to etch away the copper. After all the copper has been etched, the remaining PMMA/graphene laminated body is transferred to a bath of pure water and floated. If necessary, the transfer to the pure water bath is repeated to wash the graphene. Thereafter, the laminated body of PMMA and graphene floating on pure water is scooped up with a desired substrate. After drying, the PMMA present on the outermost surface of the laminated body is dissolved and removed using a solvent such as acetone, thereby completing the transfer of graphene onto the desired substrate. However, if one attempts to pick up the laminated body of PMMA and graphene floating on the water surface directly with tweezers or the like without scooping it up with a substrate, stress inside the PMMA film and the surface tension of water act, causing the laminated body to become wrinkled and damaging the graphene. That is, graphene using a PMMA film as a transfer support after removal of the copper substrate must be floated on the water surface before and during the transfer process, and cannot be self-supporting in the atmosphere. Thus, the existence of the pure water floating process in the transfer process was one of the main causes reducing the mass production and reproducibility of graphene production.

As prior art, laminated structures enabling simpler transfer processes or simpler transfer of graphene have been proposed.

For example, Non-Patent Document 1 reports a method of transferring graphene using a commercially available laminator and a polyvinyl alcohol (PVA) film. In this method, a graphene film produced by a CVD method on a copper foil and a PVA film are laminated together, and the laminated body is passed through a laminator to bond the graphene and the PVA film. Then, by mechanically peeling the PVA film from the copper foil, the graphene is peeled off together with the PVA film. The laminated body of graphene and PVA thus obtained is laminated onto a desired substrate (SiO₂/Si substrate in the document) and passed through a laminator, thereby transferring the graphene. Thereafter, the PVA film is dissolved and removed with hot water. This method does not involve a step of scooping up a laminated body floating on water, but since the graphene is mechanically peeled from copper, it is difficult to avoid damage to graphene. As another method, a method using electrochemical transfer and functional adhesive tapes has been proposed. Non-Patent Document 2 discloses producing a laminated body of UV tape coated with a UV-curable adhesive on polyolefin, graphene, and copper foil by contacting the UV tape with graphene on copper and irradiating UV light. By electrochemically peeling the UV tape/graphene laminated body from the copper foil, a laminated body capable of transfer without floating on water can be produced. By attaching this laminated body to a desired substrate and heating it to 90 °C, the tape is peeled off from the graphene, completing the transfer. This transfer method is a transfer method that does not involve dissolution of a support material, and the process of peeling the UV tape from the graphene film is a type of mechanical peeling.

Thus, conventional methods for transferring graphene have required a step of floating on water, or used mechanical peeling methods that tend to damage graphene, or left residues of the transfer material on graphene. Accordingly, there has been a need for a method capable of transferring high-quality CVD graphene to a desired substrate easily and without damage, without the need for floating on water.

### Prior Art Documents

### Non-Patent Documents

Non-Patent Document 1: A. Shivayogimath et al., "Do-It-Yourself Transfer of Large-Area Graphene Using an Office Laminator and Water," Chemistry of Materials, 31, pp. 2328-2336 (2019).
Non-Patent Document 2: M. Nakatani et al., "Ready-to-transfer two-dimensional materials using tunable adhesive force tapes," Nature Electronics, 7, 119-130.

### Summary of Invention

### Problems to be Solved by the Invention

An object of the present invention is to provide a method capable of easily transferring a high-quality two-dimensional layered material such as graphene onto a desired substrate without requiring floating on a water surface and without causing damage during the transfer process, as well as to provide a laminated body used for the method.

### Means for Solving the Problems

As described above, when a laminated body consisting of a PMMA film as a transfer support and a graphene film is picked up from a water surface into the atmosphere with tweezers or the like, irreversible deformation occurs in the graphene. Other damages to graphene observed in conventional transfer processes are mainly caused by local concentration of external force in mechanical peeling methods or by tensile stress inside the transfer support material. When a polymer serving as a transfer support is cast onto a graphene film on a metal catalyst, shrinkage of the polymer volume occurs as the solvent evaporates, and because one surface of the transfer support is constrained by the graphene/copper foil, tensile stress is generated in the transfer support. Furthermore, when a heating process is applied for adhesion or other purposes, deformation of the polymer also occurs, and if such deformation arises at the contact points with the graphene, it becomes a cause of damage to the graphene. The tensile stress of the transfer support film applies a compressive force to the substrate (graphene/copper foil) that constrains the transfer support. That is, compressive stress arises in the graphene/copper foil. After etching away the copper, in a laminated body consisting of the transfer support and the graphene film, there remains no support to restrain deformation of the transfer support film other than the single-atomic-layer of graphene, so the graphene is subjected to large compressive stress and easily becomes wrinkled. In conventional methods, the laminated body is under stress, and the water-air interface is used to maintain the planar structure of the laminated body. In other words, it is considered that the hydrophobic nature of PMMA/graphene and the large surface tension of water counteract the shrinkage force of the transfer support film.

To avoid the process of floating on water required in conventional PMMA transfer methods, it suffices that the laminated structure consisting of a transfer support and graphene can be self-supporting in the atmosphere without irreversible deformation. Furthermore, in such a laminated structure, it is necessary that the graphene has substantially no tearing, and that no damage occurs to the graphene even in the subsequent process of removing the transfer support.

In order to solve the above problems, the present inventors attempted to fabricate a laminated body consisting of a porous membrane and graphene. Unexpectedly, after etching the catalytic copper foil, the laminated body was capable of being self-supporting in the atmosphere while maintaining a planar structure, and the graphene in the laminated body exhibited an extremely small amount of tearing. Furthermore, by attaching this laminated body onto a desired substrate and subsequently dissolving the porous membrane, high-quality graphene could be transferred to the desired substrate without the water-floating step and with little damage.

The present invention has been completed based on the above findings and includes the following embodiments:
One aspect of the present invention is:
[1] A laminated body comprising a two-dimensional layered material and a porous membrane laminated on the two-dimensional layered material,
   wherein the laminated body is self-supporting while maintaining a planar structure in the atmosphere.

In one embodiment of the laminated body of the present invention:
[2] The laminated body according to [1],
wherein the laminated body is in a dried state.

In one embodiment of the laminated body of the present invention:
[3] The laminated body according to [1] or [2],
wherein the porous membrane has pores with an average pore diameter of 20 nm or more.

In one embodiment of the laminated body of the present invention:
[4] The laminated body according to any one of [1] to [3],
wherein the pore area ratio relative to the membrane surface of the porous membrane is 20% or more.

In one embodiment of the laminated body of the present invention:
[5] The laminated body according to any one of [1] to [4],
wherein the porous membrane has a film thickness of 100 nm or more.

In one embodiment of the laminated body of the present invention:
[6] The laminated body according to any one of [1] to [5],
wherein the porous membrane is a membrane made of a material selected from the group consisting of nitrocellulose, cellulose acetate, polyethersulfone, polytetrafluoroethylene, polyamide, polyvinylidene fluoride, regenerated cellulose, polycarbonate, polypropylene, polyvinylidene chloride, aluminum oxide, glass fibers, quartz fibers, polymethyl methacrylate, polystyrene, polyethylene, polyethylene terephthalate, and ceramics, or a mixed membrane made of two or more materials selected from the group.

In one embodiment of the laminated body of the present invention:
[7] The laminated body according to any one of [1] to [6],
wherein the porous membrane is a membrane made of a material selected from the group consisting of nitrocellulose, cellulose acetate, polycarbonate, polyvinylidene chloride, polystyrene, and polymethyl methacrylate, or a mixed membrane made of two or more materials selected from the group.

Another aspect of the present invention is:
[8] A method of transferring the laminated body according to any one of [1] to [7] to a desired substrate, comprising:
(a) attaching the two-dimensional layered material side of the laminated body to a desired position of the desired substrate.

In one embodiment of the transfer method of the present invention:
[9] The method according to [8], further comprising:
(b) removing the porous membrane of the laminated body using a solvent, after step (a). Another aspect of the present invention is:

[10] A method for producing a laminated body according to any one of [1] to [7], comprising a two-dimensional layered material and a porous membrane laminated on the two-dimensional layered material, comprising:
(i) forming the two-dimensional layered material on a metal catalyst substrate by a chemical vapor deposition (CVD) method;
(ii) further forming a porous membrane on the two-dimensional layered material to prepare a laminated body; and
(iii) removing the metal catalyst substrate by etching.

In one embodiment of the method for producing a laminated body of the present invention:
[11] The method according to [10],
wherein, in step (ii), the porous membrane is formed on the two-dimensional layered material by a phase inversion method.

In one embodiment of the method for producing a laminated body of the present invention:
[12] The method according to [10] or [11],
wherein, in step (ii), the solvent used to dissolve a polymer for forming the porous membrane is a mixed solvent comprising a good solvent and a poor solvent for the polymer.

### Effects of the Invention

According to the laminated body of the present invention, a high-quality two-dimensional layered material can be easily transferred to a desired substrate without requiring floating on a water surface and without causing damage during the transfer process.

Since the laminated body of the present invention can be self-supporting in the atmosphere, storage and transportation can also be facilitated.

### Brief Description of Drawings

**[****Fig. 1]** Fig. 1 is a schematic diagram showing one embodiment of the laminated body according to the present invention. The laminated body comprises a porous membrane and a two-dimensional layered material.
**[****Fig. 2A]** Fig. 2A is a scheme illustrating one embodiment of the transfer method of the laminated body according to the present invention. Fig. 2A shows a method in which a substrate 11 is placed over a laminated body (porous membrane/two-dimensional layered material) stacked on a membrane, thereby transferring the laminated body 1 onto the substrate 11.
**[****Fig. 2B]** Fig. 2B is a scheme illustrating one embodiment of the transfer method of the laminated body according to the present invention. Fig. 2B shows a method of transferring a laminated body stacked on a membrane onto a substrate.
**[****Fig. 2C]** Fig. 2C is a scheme illustrating one embodiment of the transfer method of the laminated body according to the present invention. Fig. 2C shows a method of directly transferring the laminated body onto a substrate without using a membrane.
**[****Fig. 2D]** Fig. 2D is a schematic diagram showing exposure of the laminated body to acetone vapor to dissolve and remove the porous membrane.
**[****Fig. 3A]** Fig. 3A is a scheme illustrating one embodiment of the method for producing the laminated body according to the present invention.
**[****Fig. 3B]** Fig. 3B is a scheme illustrating a conventional method for producing a graphene electrode using PMMA as a transfer support film.
**[****Fig. 4]** Fig. 4 shows SEM images of the porous membrane surface of a porous membrane/graphene laminated body produced in Example 1 below.
**[****Fig. 5]** Fig. 5 shows histograms of pore diameters present on the porous membrane surface of a porous membrane/graphene laminated body produced in Example 1 below.
**[****Fig. 6]** Fig. 6 shows histograms of pore diameters present on the porous membrane surface of a porous membrane/graphene laminated body produced in Example 1 below.
**[****Fig. 7]** Fig. 7(a) shows a photograph of a PMMA film/graphene film laminated body scooped up on a membrane and dried, performed in Example 3 below.
Fig. 7(b) shows a photograph of a mixed cellulose ester/graphene film laminated body scooped up on a membrane and dried, performed in Example 3 below.
**[****Fig. 8]** Fig. 8 shows SEM images of the surface of a mixed cellulose ester membrane produced in Example 5 below.
Fig. 8(a) shows an SEM image of a mixed cellulose ester membrane produced using acetone as a solvent, and
Fig. 8(b) shows an SEM image of a mixed cellulose ester membrane produced using an acetone/formamide mixed solvent.
**[****Fig. 9]** Fig. 9 shows SEM images of the porous membrane surface of six types of porous membrane/graphene laminated bodies produced by a solution casting method in Example 5 below.
   The conditions of mixed cellulose ester solution concentration and immersion in ultrapure water in Figs. 18(a)-(f) are as follows:
   (a) 4 wt% without immersion;
   (b) 4 wt% with immersion;
   (c) 8 wt% without immersion;
   (d) 8 wt% with immersion;
   (e) 12 wt% without immersion;
   (f) 12 wt% with immersion.

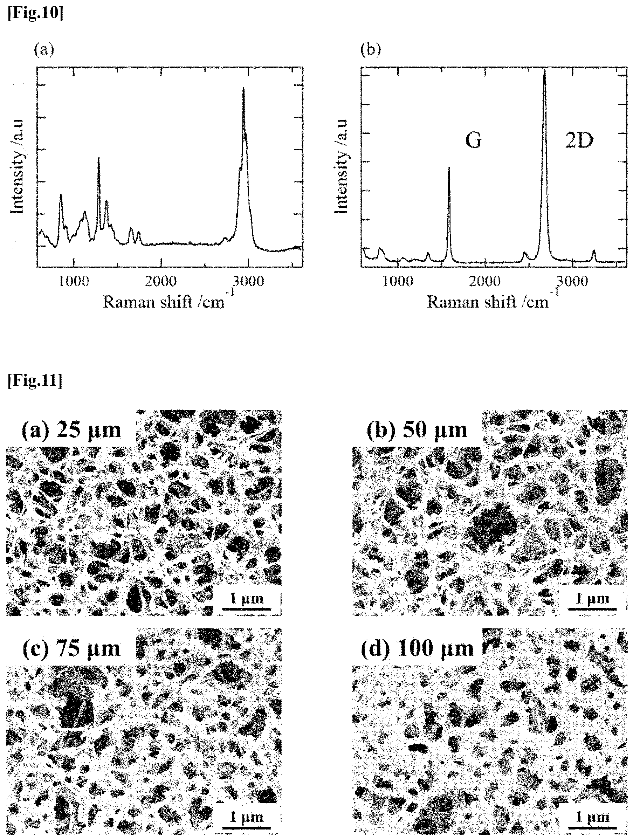

**[****Fig. 10]** Fig. 10(a) shows a Raman spectrum of a porous mixed cellulose ester membrane/graphene film/quartz glass substrate laminated body produced in Example 5 below.
Fig. 10(b) shows a Raman spectrum of a graphene film on a quartz glass substrate after removal of the porous mixed cellulose ester membrane.
**[****Fig. 11]** Fig. 11 shows SEM images of the mixed cellulose ester membrane in a laminated body composed of a porous mixed cellulose ester membrane/graphene film/copper foil produced in Example 6 below.
Fig. 11(a) shows an SEM image when the applicator gap height during production of the mixed cellulose ester membrane in the laminated body was 25 µm.
Figs. 11(b)-(d) show SEM images when the applicator gap heights during production of the mixed cellulose ester membrane in the laminated body were 50 µm, 75 µm, and 100 µm, respectively.

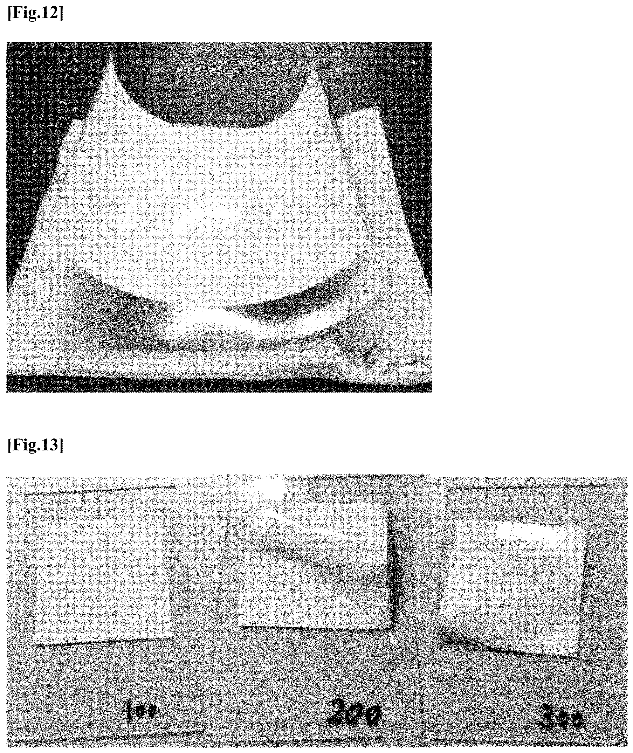

**[****Fig. 12]** Fig. 12 shows a photograph of a mixed cellulose ester membrane produced in Example 7 below, prepared using an applicator gap height of 300 µm.
**[****Fig. 13]** Fig. 13 shows photographs of mixed cellulose ester membranes produced in Example 7 below (applicator gap heights of 100 µm, 200 µm, or 300 µm) transferred onto a glass slide.

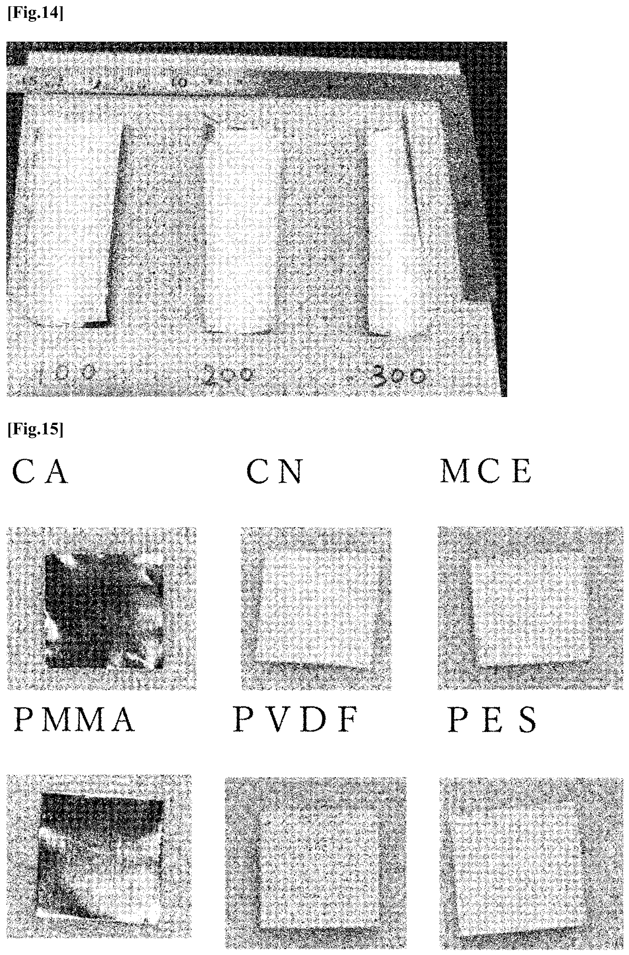

**[****Fig. 14]** Fig. 14 shows a photograph of mixed cellulose ester membranes formed on graphene grown on copper foil, prepared in Example 7 below (applicator gap heights of 100 µm, 200 µm, or 300 µm).
**[****Fig. 15]** Fig. 15 shows photographs of laminated bodies produced in Example 8 below, in which one of six different porous membranes was formed on a copper foil/graphene film.

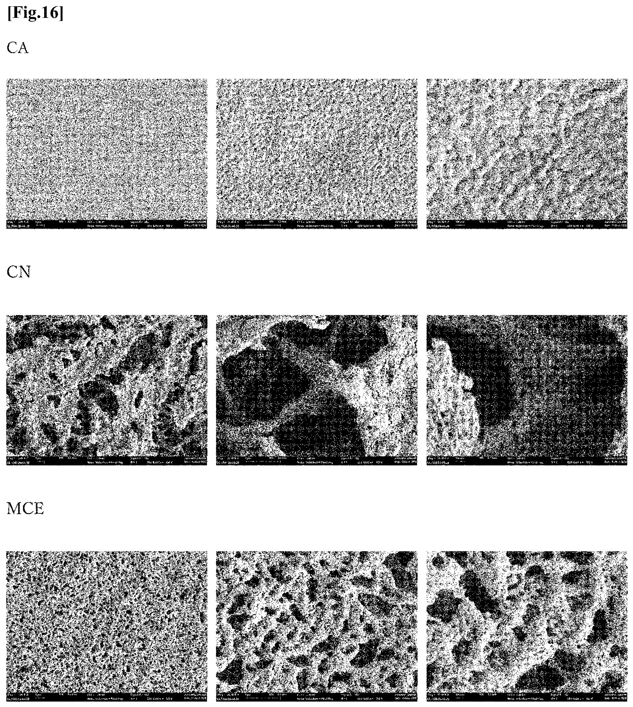

**[****Fig. 16]** Fig. 16 shows SEM images of the surfaces of porous membranes formed on copper foil/graphene films in Example 8 below. The porous membranes are composed of cellulose acetate, nitrocellulose, or mixed cellulose ester.

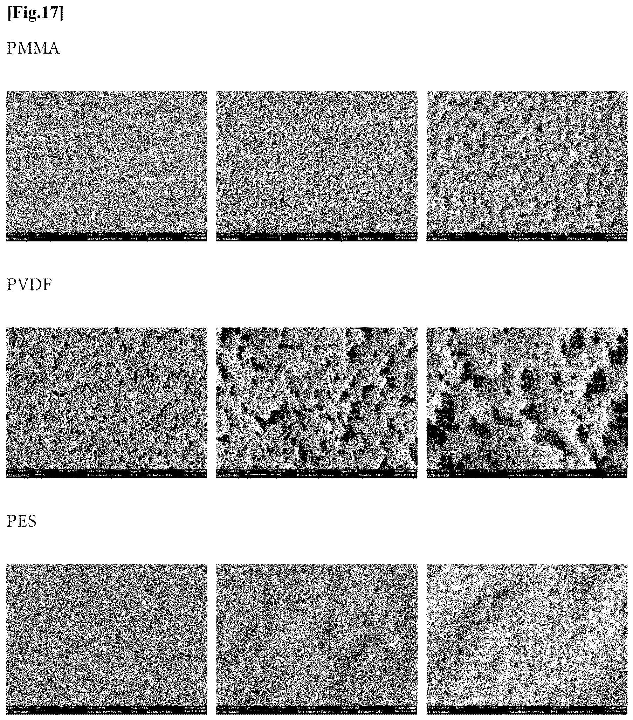

**[****Fig. 17]** Fig. 17 shows SEM images of the surfaces of porous membranes formed on copper foil/graphene films in Example 8 below. The porous membranes are composed of PMMA, PVDF, or PES.

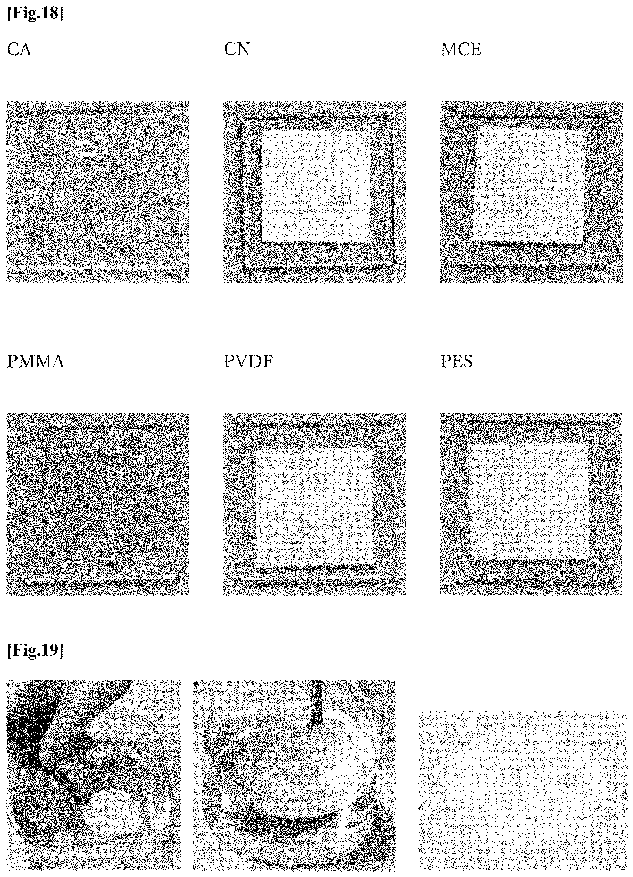

**[****Fig. 18]** Fig. 18 shows photographs of laminated bodies composed of a porous membrane/graphene film/quartz glass substrate produced in Example 9 below.
**[****Fig. 19]** Fig. 19 shows images of the process of recovering the laminated body from the etchant in the production method carried out in Example 10 below. The left image shows the step of recovering the laminated body using a membrane filter, in which the filter is brought into contact with the support-film side of the laminated body floating on the surface of the etchant and the laminated body is lifted up. The center image shows the step of washing the laminated body attached to the membrane filter in a water bath. The right image shows the step of drying the laminated body on the membrane filter with the graphene side facing upward.

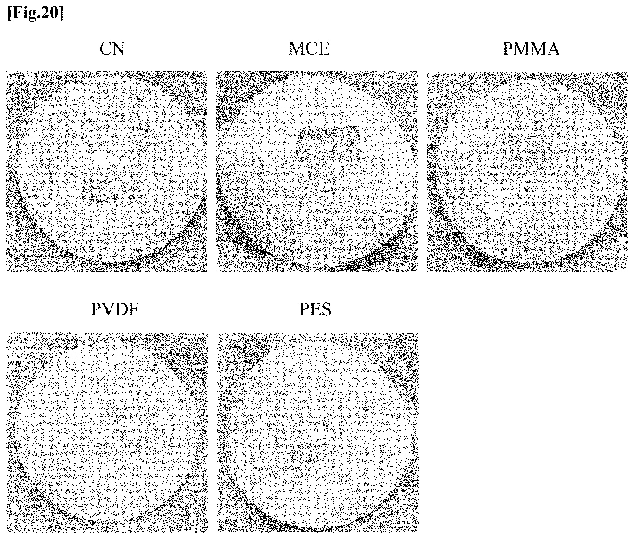

**[****Fig. 20]** Fig. 20 shows photographs of porous membrane/graphene film laminated bodies produced in Example 10 below, the laminated bodies being placed on a membrane filter. The porous membranes used were CN, MCE, PMMA, PVDF, and PES.

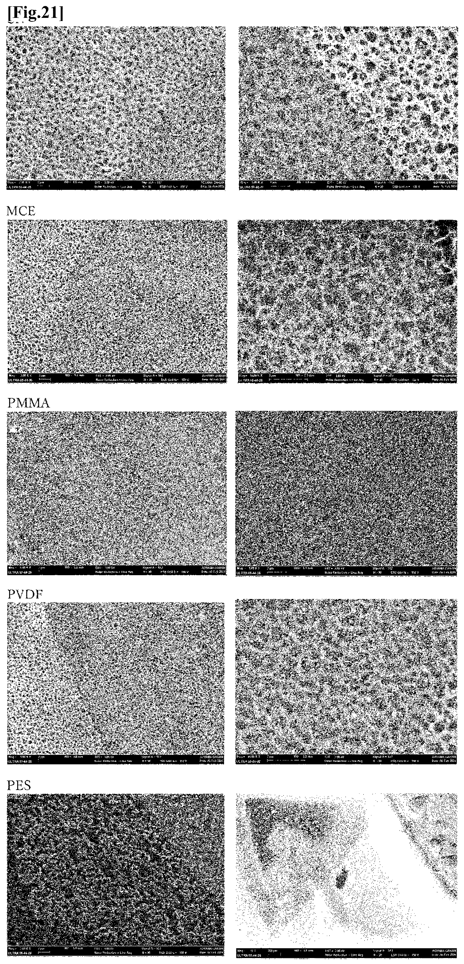

**[****Fig. 21]** Fig. 21 shows SEM images of the graphene surface of the porous membrane/graphene film laminated bodies produced in Example 10 below. The porous membranes used were CN, MCE, PMMA, PVDF, and PES.

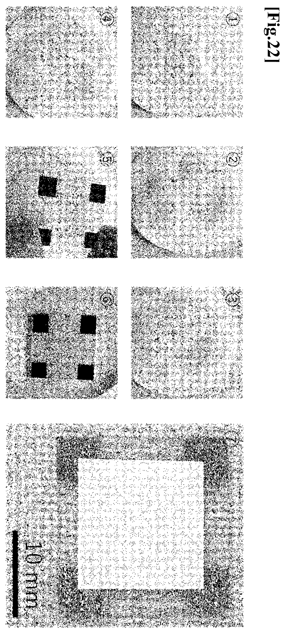

**[****Fig. 22]** Fig. 22 shows photographs of each step when transferring a self-supporting graphene/porous membrane laminated body onto a quartz glass substrate in Example 11 below. Fig. 20(1) shows the self-supporting laminated body placed on a membrane filter; Figs. 22(2) and 22(3) show the membrane filter underlying the porous membrane wetted with pure water; Fig. 22(4) shows pure water dropped onto the graphene film of the laminated body; and Figs. 22(5) and 22(6) show a 20 mm × 20 mm quartz glass substrate with gold/chromium (5 mm × 5 mm) deposited at the four corners placed over the laminated body.

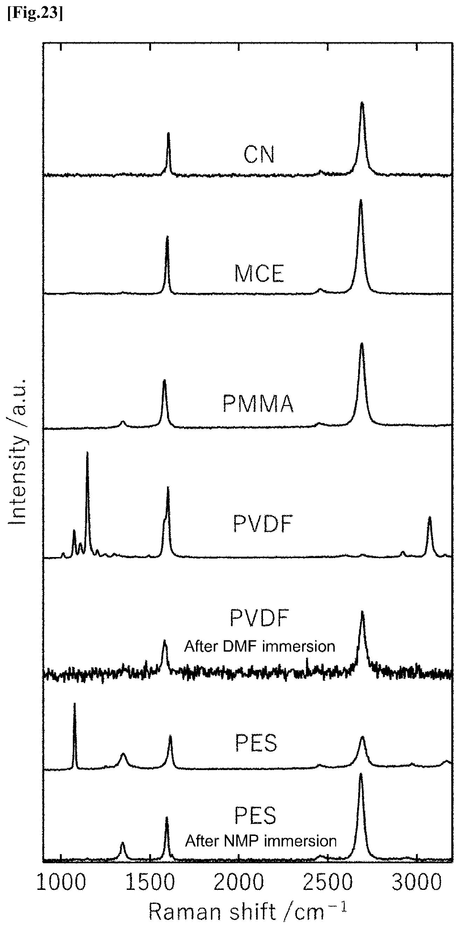

**[****Fig. 23]** Fig. 23 shows Raman spectra of the graphene films produced on the quartz glass substrates in Example 11 below.

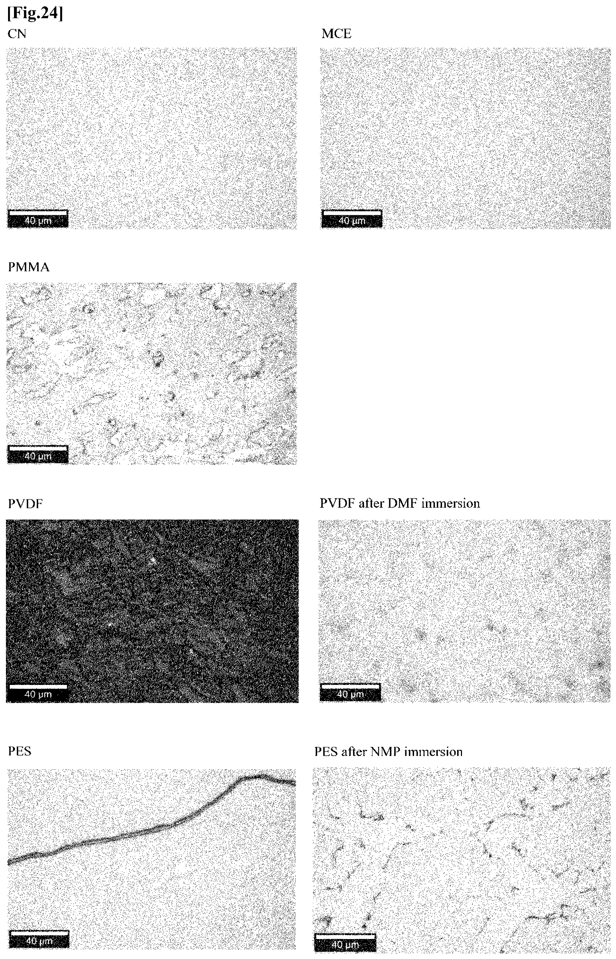

**[****Fig. 24]** Fig. 24 shows optical microscope images of the graphene film on the quartz glass substrate produced in Example 11 below, taken during Raman spectroscopy.

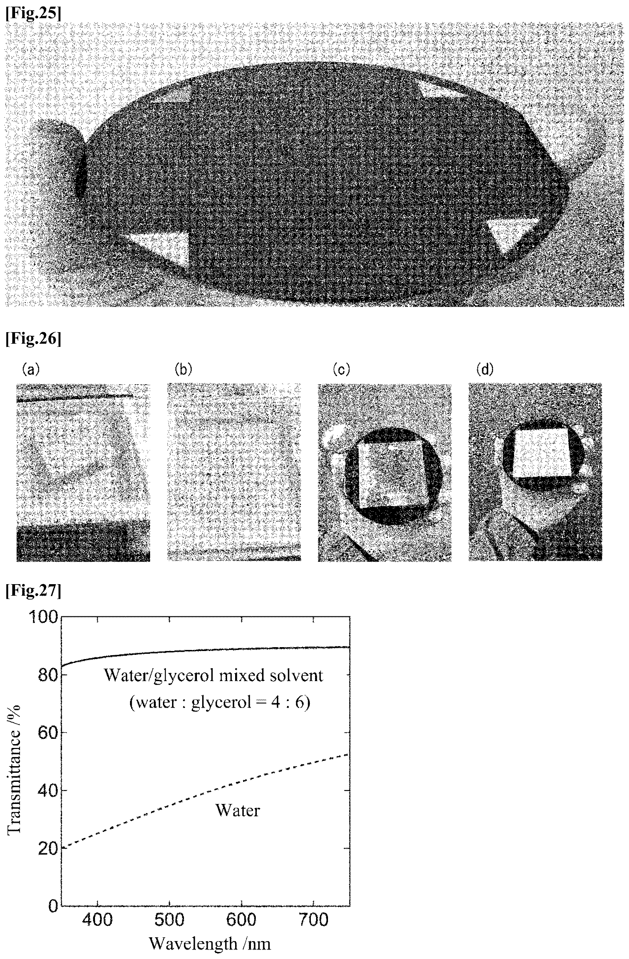

**[****Fig. 25]** Fig. 25 shows a photograph of a monolayer graphene film produced on an SiO₂/Si substrate in Example 12 below.
**[****Fig. 26]** Fig. 26 shows photographs of each step in the transfer process conducted in Example 12 below.
**[****Fig. 27]** Fig. 27 shows the transmittance spectra of a PVDF/graphene/quartz glass substrate laminated body in water and water/glycerol solutions, measured in Example 13 below.

### Modes for Carrying Out the Invention

One aspect of the present invention provides a laminated body comprising a two-dimensional layered material and a porous membrane laminated on the two-dimensional layered material, wherein the laminated body is self-supporting while maintaining planarity in the atmosphere.

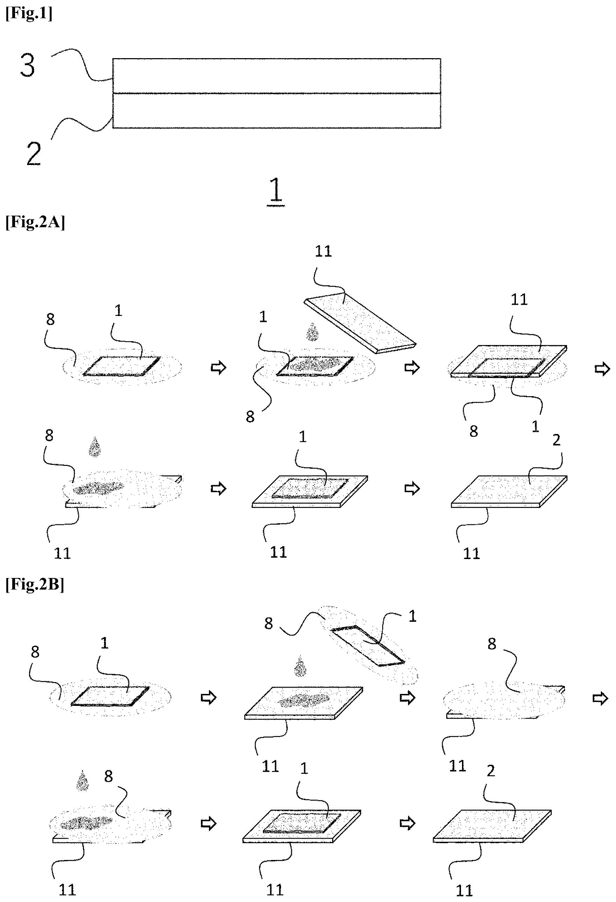

An embodiment of the laminated body according to this aspect is shown in Fig. 1. As shown in Fig. 1, the laminated body 1 is composed of a two-dimensional layered material 2 and a porous membrane 3.

In the porous membrane constituting the laminated body of the present invention, since pores are uniformly distributed within the membrane, internal stress is prevented from being locally concentrated and is instead evenly dispersed. By dispersing the internal stress through the internal voids, the stress is relieved, thereby improving the overall structural stability of the laminated body. In addition, under external pressure, the pores deform and disperse the stress, so that the porous membrane itself has a structure resistant to deformation. In the laminated body of the present invention, the porous membrane is used as a transfer support film.

Furthermore, porous membranes such as mixed cellulose ester membranes or nitrocellulose, which can be used in the laminated body of the present invention, are readily soluble in solvents such as acetone. Therefore, it is possible to obtain a transferred film with minimal polymer residue. Moreover, since the transfer support material is a porous membrane, during the process of dissolving the transfer support material, the solvent can readily penetrate into the transfer support material, making it possible to remove the transfer support material rapidly and almost completely from the two-dimensional layered material.

In this specification, the term "two-dimensional layered material" refers to a material capable of forming atomically thin layers. Such two-dimensional layered materials include, but are not limited to: graphene, doped graphene, graphene oxide, hydrogenated graphene, fluorinated graphene, hexagonal boron nitride, molybdenum disulfide, vanadium oxide, silicon, covalent organic frameworks, layered transition metal dichalcogenides (e.g., MoS₂, TiS₂), two-dimensional oxides (e.g., graphene oxide, NiO₂), layered group IV and group III metal chalcogenides (e.g., SnS, PbS, GeS), silicene, germanene, and layered binary compounds of group IV elements and group III-V elements (e.g., SiC, GeC, SiGe).

The two-dimensional layered material may be a monolayer or may be formed of multiple layers. When a multilayer two-dimensional layered material is used as a transparent conductive film, it is preferably composed of two to three layers from the viewpoint of light transmittance, but the invention is not limited thereto.

The two-dimensional layered material is preferably a continuous film. The term "continuous film" refers to a film in which there are no pores or tears and the components constituting the film are continuously connected. A two-dimensional layered material in the form of a continuous film has a uniform surface and can reduce sheet resistance, and is therefore preferred.

In this specification, that the two-dimensional layered material is a continuous film means that the two-dimensional layered material in the laminated body is formed and attached as a continuous film on the surface of the porous membrane. When the two-dimensional layered material is transferred as a pattern onto the surface of the porous membrane, each two-dimensional layered material constituting the pattern is attached as a continuous film on the surface of the porous membrane. The laminated body of the present invention can transfer a two-dimensional layered material as a continuous film onto a desired substrate. That the two-dimensional layered material is a continuous film means that, when the two-dimensional layered material is transferred to a specific region of the porous membrane surface (for example, the entire surface or a part thereof), the coverage of the two-dimensional layered material in the specific region is 95% or more. In preferred embodiments, the coverage of the two-dimensional layered material is 96% or more, 97% or more, 98% or more, 99% or more, or 99.5% or more.

As a method for measuring the coverage, a laminated body consisting of a two-dimensional layered material and a porous membrane may be transferred onto an SiO₂/Si substrate, and after removal of the porous membrane, an optical microscope image of the two-dimensional layered material may be taken, and the coverage is calculated from the color ratio of the monolayer portion. Raman mapping may also be used in combination.

In this specification, when the two-dimensional layered material is described as being a monolayer, it may partially include multilayer two-dimensional layered material, but is substantially formed as a monolayer. The term "substantially formed as a monolayer" means, for example, that the area of multilayer two-dimensional layered material present on the two-dimensional layered material is 15% or less, 10% or less, 5% or less, or 3% or less. When the area of multilayer two-dimensional layered material on the two-dimensional layered material is 3% or less, the coverage of monolayer two-dimensional layered material within the two-dimensional layered material is 97% or more.

In preferred embodiments, the two-dimensional layered material is graphene. When the two-dimensional layered material is graphene, the graphene film may be doped by substituting carbon atoms of the graphene with nitrogen, boron, or the like, or by molecular adsorption. In a multilayer graphene film, metal chlorides or the like may be intercalated between the layers.

Methods for forming the two-dimensional layered material or graphene film are known to those skilled in the art (see, for example, T. Watanabe et al., "Single-layer graphene as a transparent electrode for electrogenerated chemiluminescence biosensing," Electrochemistry Communications, 138, 107290 (2022). https://doi.org/10.1016/j.elecom.2022.1 [Reference 1]), and may preferably be formed by a chemical vapor deposition (CVD) method. As long as the laminated body 1 functions, graphene films formed by known methods such as mechanical exfoliation, chemical exfoliation, thermal decomposition of SiC, or reduction of graphene oxide may also be used.

The porous membrane 3 is laminated on a first surface of the two-dimensional layered material 2. The porous membrane 3 may be laminated so as to cover the entire surface of the two-dimensional layered material 2, or only a part thereof. Preferably, the porous membrane 3 is capable of supporting the two-dimensional layered material such that, after the metal catalyst substrate on which the two-dimensional layered material has been grown is removed by etching for transfer to a desired substrate, the two-dimensional layered material can maintain its shape in the atmosphere.

The thickness of the porous membrane 3 is preferably 100 nm or more. If the thickness is less than 100 nm, the supporting force for the two-dimensional layered material formed by the CVD method becomes insufficient, and tearing is likely to occur during transfer. In preferred embodiments, the lower limit of the thickness of the porous membrane 3 is 100 nm or more, and more preferably 1 µm or more. In further preferred embodiments, the thickness is 5 µm or more. In preferred embodiments, the upper limit of the thickness of the porous membrane 3 is 300 µm or less, and more preferably 100 µm or less. If the membrane is too thick, even with a porous structure, the residual stress of the support material becomes large, impairing the quality of the two-dimensional layered material after transfer.

Examples of the porous membrane 3 that can be used in the present invention include, but are not limited to, membranes made of materials selected from the group consisting of nitrocellulose, cellulose acetate, polyethersulfone, polytetrafluoroethylene, polyamide, polyvinylidene fluoride, regenerated cellulose, polycarbonate, polypropylene, polyvinylidene chloride, aluminum oxide, glass fibers, quartz fibers, polymethyl methacrylate, polystyrene, polyethylene, polyethylene terephthalate, and ceramics, or mixed membranes made of two or more materials selected from the group.

From the viewpoint of ease of dissolution in solvents, membranes made of materials selected from the group consisting of nitrocellulose, cellulose acetate, polycarbonate, polyvinylidene chloride, polystyrene, and polymethyl methacrylate, or mixed membranes made of two or more materials selected from the group, may be exemplified. Furthermore, the porous membrane 3 may also be a laminate of two or more porous membranes.

As a mixed membrane made of two or more materials, a mixed cellulose ester, which is a mixture of cellulose acetate and nitrocellulose, can be suitably exemplified. For example, when using a mixed membrane of cellulose acetate and nitrocellulose, the weight ratio of cellulose acetate to nitrocellulose in the mixed solution used to form the porous membrane is preferably in the range of 1:1 to 1:3, and more preferably 1:2 or greater. Preferred examples of the porous membrane 3 include nitrocellulose, cellulose acetate, and mixed cellulose ester.

After the laminated body is transferred onto a desired substrate, the porous membrane can be dissolved with a solvent. Therefore, in preferred embodiments, the porous membrane is a membrane made of a material that is easily soluble in a solvent. Examples of such porous membranes include nitrocellulose, cellulose acetate, mixed cellulose ester, polymethyl methacrylate, and polystyrene. By using a porous membrane that is easily soluble in a solvent, damage to the two-dimensional layered material during removal of the porous membrane after transfer can be suppressed, and it is also preferable in that no residue of the porous membrane remains on the two-dimensional layered material film.

Although not limited thereto, the average pore diameter of the porous membrane 3 is preferably 20 nm to 20 µm. If it is less than 20 nm, the stress relaxation function deteriorates, which is undesirable. If it exceeds 20 µm, the density of the porous membrane becomes low, the supporting force during transfer of the two-dimensional layered material weakens, and tearing of the film is likely to increase, which is undesirable. In preferred embodiments, the average pore diameter of the porous membrane 3 is 200 nm to 10 µm. The average pore diameter of the porous membrane 3 refers to the average value of the pore diameters of the pores present on the surface of the porous membrane. The average value of pore diameters can be measured, for example, by observing the surface of the porous membrane with a scanning electron microscope (SEM) or the like and measuring the area of several hundred (for example, 300) randomly selected pores. From the area of each pore, the diameter is calculated assuming the pore is circular, and the average of these values can be taken as the surface average pore diameter.

Although not limited thereto, the porous membrane 3 preferably has a pore area ratio of 20% or more relative to the interfacial area between the two-dimensional layered material and the porous membrane. If the pore area ratio relative to the interfacial area between the two-dimensional layered material and the porous membrane is less than 20%, the stress relaxation function deteriorates, making the two-dimensional layered material more susceptible to damage, which is undesirable. The upper limit of the pore area ratio relative to the interfacial area between the two-dimensional layered material and the porous membrane is not particularly restricted as long as the porous membrane 3 functions as a transfer support film for the two-dimensional layered material 2, but may be, for example, 80% or less. In preferred embodiments, the pore area ratio relative to the interfacial area between the two-dimensional layered material and the porous membrane is 25% or more, and in more preferred embodiments, the pore area ratio relative to the membrane surface is 30% or more. The pore area ratio relative to the interfacial area between the two-dimensional layered material and the porous membrane of the porous membrane 3 refers to the ratio of the total area of all pores present on one surface of the porous membrane to the geometric area of that surface. A person skilled in the art can measure the pore area by known methods. For example, when the two-dimensional layered material is graphene, the pore area can be measured by observing the structure of the porous membrane at the graphene/porous membrane interface from the graphene side using SEM or the like.

In one embodiment, when the two-dimensional layered material is a continuous monolayer graphene film, the sheet resistance of the monolayer graphene film in the laminated body according to the present invention can be 500 Ω/sq or less. In more preferred embodiments, the electrical resistance of the monolayer graphene film is 400 Ω/sq or less, or 300 Ω/sq or less. In still more preferred embodiments, the electrical resistance of the monolayer graphene film is 200 Ω/sq or less. The sheet resistance is defined as the electrical resistance of the film as an actual conductive film, which is given by resistivity divided by film thickness. The electrical resistance of the laminated body can be measured by the van der Pauw method.

Another aspect of the present invention provides a method of transferring the above laminated body onto a desired substrate. The transfer method includes the following step: (a) attaching the two-dimensional layered material side of the laminated body to a desired position on the desired substrate.

According to the transfer method of the present invention, it is not necessary to float the two-dimensional layered material on a water surface, and the two-dimensional layered material can be easily transferred to a desired position of a desired substrate.

The transfer method of the present invention includes step (a) of attaching the two-dimensional layered material side of the laminated body to a desired position on a desired substrate.

The term "desired substrate" is not particularly limited as long as the two-dimensional layered material can adhere to it and the substrate has resistance to the solvent used to dissolve the porous membrane. Examples of such substrates include transparent insulating substrates and SiO₂/Si substrates. The shape of the substrate is not limited to a plane, and the substrate may have a curved surface. In addition, since the transfer method of the present invention does not require floating the laminated body on a water surface during the transfer step, the substrate may be one fixed in a building, such as window glass (for example, fixed in a vertical direction).

Examples of transparent insulating substrates that can be used in the present invention include, but are not limited to, quartz, glass, other transparent glass materials, sapphire glass, polyethylene terephthalate, polyester, acrylic resin, polyethylene, nylons, polyvinyl chloride, and polyimide.

The term "desired position" refers to a position on a desired substrate to which the two-dimensional layered material is to be transferred. In conventional transfer methods, it was necessary to float the laminated body on a water surface and scoop it up with a desired substrate, making precise adjustment of the transfer position difficult. In contrast, according to the transfer method of the present invention, since the laminated body itself can be self-supporting in the atmosphere, the two-dimensional layered material can be easily transferred to a desired position on a substrate. Furthermore, according to the transfer method of the present invention, a large-area two-dimensional layered material, which was difficult to transfer conventionally, can be transferred onto a desired substrate. The term "large area" for the two-dimensional layered material refers to, for example, an area of 5 cm² or more. In preferred embodiments, the graphene film after transfer using the laminated body of the present invention has an area of 10 cm² or more, and more preferably 25 cm² or more.

To attach the two-dimensional layered material side of the laminated body to a desired position on a desired substrate, it is sufficient to drop a small amount of a solvent such as water in advance onto the two-dimensional layered material side and/or the substrate, and then bring the laminated body into contact with the substrate. After the laminated body and the substrate are brought into contact, it is preferable to allow the solvent, such as water, to dry either naturally or artificially. The term "artificial drying" includes, but is not limited to, heating or blowing air using an incubator, a blower, or the like.

The solvent used for adhesion between the laminated body and the substrate is not particularly limited as long as it can promote adhesion due to van der Waals forces generated between the laminated body and the substrate. Preferably, such a solvent does not have the property of dissolving the porous membrane and is volatile. Examples include, but are not limited to, water, hexane, heptane, isobutanol, diethyl ether, and toluene.

After the laminated body is transferred to a desired substrate, the laminated body may be used without removing the porous membrane, or it may be used as a two-dimensional layered material after removing the porous membrane. When the laminated body consisting of the two-dimensional layered material and the porous membrane is used without removing the porous membrane, it is preferable that the laminated body is transparent depending on the intended device (substrate). For this purpose, the voids of the porous membrane may be filled with the material constituting the porous membrane, or with another material having the same refractive index as the material constituting the porous membrane. If the porous membrane has no absorption band in the visible light wavelength region, the laminated body becomes transparent.

As a method for filling the voids of the porous membrane with another material having the same refractive index as the material constituting the porous membrane, a method of filling the voids of the porous membrane with a solvent having a refractive index matching that of the porous membrane can be employed. A person skilled in the art can suitably prepare or adjust such a solvent. For example, the solvent may be, but is not limited to, a combination of solvents having different refractive indices, such as glycerol and water, with the composition ratio adjusted so that the refractive index matches that of the porous membrane. The solvent to be used is not particularly limited as long as it does not interfere with the use of the laminated body as an electrode.

In one embodiment of the transfer method of the present invention, after step (a), step (b) of removing the porous membrane of the laminated body using a solvent may be included. As the solvent for dissolving the porous membrane, any known solvent capable of dissolving the porous membrane may be used, and the solvent can be appropriately selected depending on the type of porous membrane. Examples of such solvents include ketones (e.g., acetone), alcohols, acetic acid, or combinations thereof. The laminated body may be immersed in these solvents, or the solvents may be heated so that the laminated body is exposed to the vapor of the solvents, thereby dissolving and removing the porous membrane.

With respect to the transfer method of the laminated body according to the present invention, a scheme of one embodiment is shown in Figs. 2A-2C.

Fig. 2A shows a method of transferring the laminated body 1 (porous membrane/two-dimensional layered material) stacked on a membrane 8 onto a substrate 11 by placing the substrate 11 over the laminated body 1. More specifically, the laminated body 1 stacked on the membrane 8 is moistened with water, and the substrate 11 is attached to the laminated body 1. The attached substrate 11, laminated body 1, and membrane 8 are then inverted upside down, and the membrane 8 is further moistened with water. Thereafter, by peeling off the membrane 8 from the laminated body 1, the laminated body 1 is transferred onto the substrate 11. Subsequently, if desired, the porous membrane 3 in the laminated body 1 can be removed, thereby transferring the two-dimensional layered material 2 onto the substrate 11.

Fig. 2B shows a method of transferring the laminated body 1 stacked on a membrane 8 onto a substrate 11. A desired position of the substrate 11 is moistened with water, and the membrane 8 carrying the laminated body 1 is attached to the substrate 11 so that the porous membrane 3 in the laminated body 1 comes into contact with the substrate 11. The membrane 8 is then further moistened with water and removed, thereby transferring the laminated body 1 onto the substrate 11. Subsequently, if desired, the porous membrane 3 in the laminated body 1 can be removed, thereby transferring the two-dimensional layered material 2 onto the substrate 11.

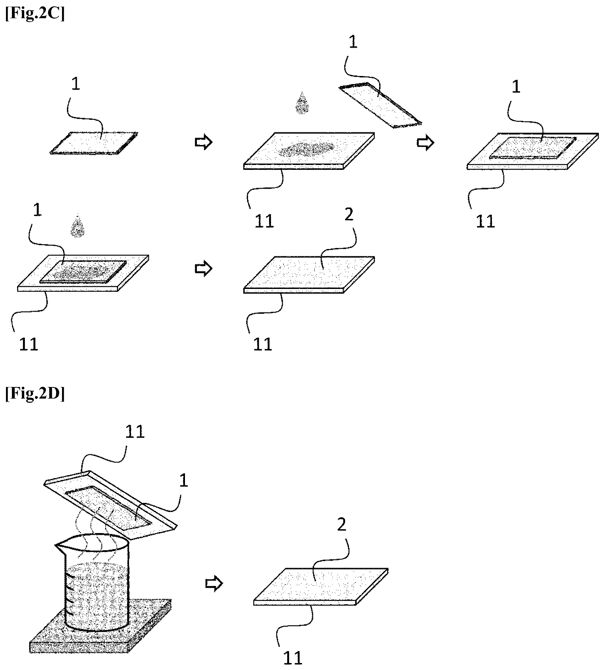

Fig. 2C shows a method of directly transferring the laminated body 1 onto a substrate 11 without using a membrane. A desired position of the substrate 11 is moistened with water, and the laminated body 1 is attached to the substrate 11 so that the porous membrane 3 in the laminated body 1 comes into contact with the substrate 11, thereby transferring the laminated body 1 onto the substrate 11. Subsequently, if desired, the porous membrane 3 in the laminated body 1 can be removed using a solvent, thereby transferring the two-dimensional layered material 2 onto the substrate 11.

Fig. 2D is a schematic diagram showing exposure of the laminated body 1 to acetone vapor to dissolve and remove the porous membrane 3.

Another aspect of the present invention provides a method for producing a laminated body comprising a two-dimensional layered material and a porous membrane. The method for producing the laminated body includes the following steps:
(i) forming the two-dimensional layered material on a metal catalyst substrate by a chemical vapor deposition (CVD) method;
(ii) further forming a porous membrane on the two-dimensional layered material to prepare a laminated body; and
(iii) removing the metal catalyst substrate by etching.

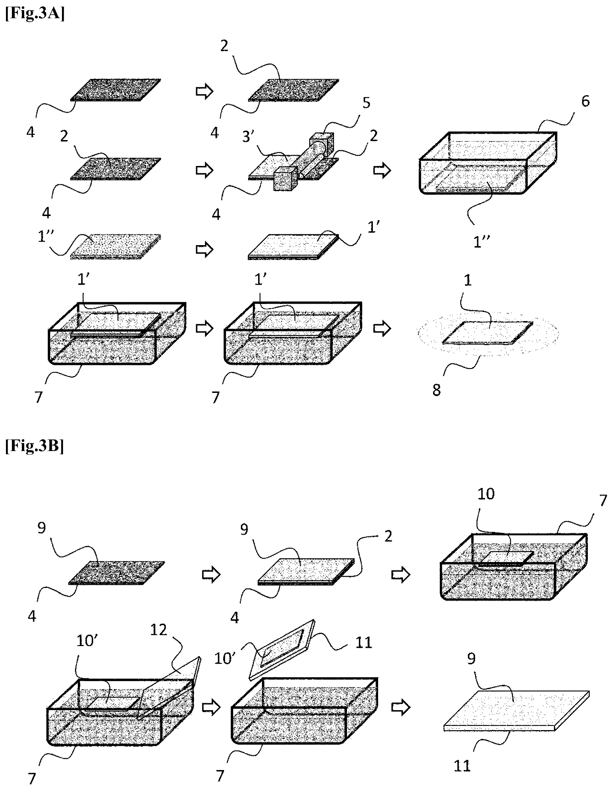

Conventionally, in producing an electrode having a graphene film as a two-dimensional layered material, a PMMA film was formed on the graphene film on a metal catalyst substrate as a transfer support film, and after etching the metal catalyst substrate and transferring to a desired substrate, a step of removing the PMMA film was included. In this method, after etching of the metal catalyst substrate, it was necessary to float the laminated body consisting of the graphene film and the transfer support film on a water surface, and the subsequent transfer to the substrate also had to be carried out as part of a continuous sequence of steps. However, according to the method for producing the laminated body of the present invention, the obtained laminated body can be self-supporting in the atmosphere, and can maintain its shape even after being scooped up from the water surface. Therefore, it is not necessary to perform etching of the metal catalyst substrate and transfer to the substrate as a continuous series of steps. That is, transfer of the laminated body to a desired substrate can be performed at a desired timing. An example of a scheme of the production method according to the present invention is shown in Fig. 3A. In the embodiment shown in Fig. 3A, a two-dimensional layered material 2 is formed on a metal catalyst substrate 4, and then a porous-membrane-forming solution 3' is applied onto the two-dimensional layered material 2 by an applicator. The resulting laminated body is transferred into a water bath containing ultrapure water to promote formation of the porous membrane. The obtained laminated body 1" (porous membrane/two-dimensional layered material/metal catalyst substrate; wet state) is dried, and the dried laminated body 1' (porous membrane/two-dimensional layered material/metal catalyst substrate) is floated on an etchant in an etching bath to etch the metal catalyst substrate. Finally, by recovering the laminated body 1 from the etchant, the laminated body can be produced. In the embodiment shown in Fig. 3A, a membrane 8 is used to recover the laminated body 1.

A conventional production scheme of a graphene electrode using a transfer support film is shown in Fig. 3B. As shown in Fig. 3B, in the conventional transfer method of a graphene film using PMMA as a transfer support film, it is necessary to scoop up the PMMA laminated body (PMMA/graphene film) directly from the etching bath 7 (and a subsequent water bath for washing) with a substrate 11. After the PMMA laminated body 10' is scooped up with the substrate 11, the PMMA is removed. In the conventional transfer method of a graphene film using PMMA as a transfer support film, PMMA residue remained on the surface of the graphene film after removal of PMMA. Such residue adversely affects the properties of the graphene film. In contrast, the production method of the present invention can use a porous membrane that is readily soluble in a solvent, and in this case, the above problem can be avoided.

The method for producing a laminated body according to the present invention includes step (i) of forming a two-dimensional layered material on a metal catalyst substrate 15 by a CVD method. Methods of forming a two-dimensional layered material 2 on a metal catalyst substrate 15 by a CVD method are known, and can be implemented, for example, with reference to Reference 1 described above. The metal catalyst substrate 15 that can be used in the present invention is not particularly limited and may be any known substrate used for forming a two-dimensional layered material 2. Preferred examples include copper substrates, nickel substrates, cobalt substrates, iridium substrates, platinum substrates, gold substrates, and alloy substrates thereof. Substrates obtained by depositing these metals on heat-resistant substrates may also be used.

After forming the two-dimensional layered material on the metal catalyst substrate in step (i), step (ii) of further forming a porous membrane on the two-dimensional layered material is carried out. The method of forming the porous membrane 3 on the two-dimensional layered material is not limited, and known methods such as spin coating, spray coating, a phase inversion method using an applicator or bar coater, and vapor deposition can be used. Below, a method of forming a porous membrane by spin coating will be described.

A polymer serving as a material for the porous membrane 3 is dissolved in a solvent to prepare a porous-membrane-forming solution. The polymer may be appropriately selected depending on the porous membrane 3 to be formed. One polymer may be used for forming the porous membrane 3, or two or more polymers may be used.

When forming the porous membrane 3 by spin coating, it is known that the film thickness and average pore diameter of the porous membrane vary depending on conditions such as the selection of solvent, the polymer concentration in the solvent, the rotational speed of the spin coater, the viscosity of the solution, and the average molecular weight of the polymer (which affects viscosity). Those skilled in the art can appropriately set the above conditions according to the polymer and solvent used so as to form a porous membrane having a desired film thickness and average pore diameter. Examples of conditions for forming the porous membrane 3 by spin coating are given below by way of illustration, but the invention is not limited thereto.

The solvent is not particularly limited as long as it can dissolve the polymer and form a porous membrane by a spin coating method. Examples of the solvent include acetone, methanol, diethyl ether, and ethyl acetate. Preferably, the solvent is a low-boiling solvent. In preferred embodiments, the solvent has a boiling point of 100 °C or lower, and in more preferred embodiments, the solvent has a boiling point of 80 °C or lower. Generally, low-boiling solvents have fast evaporation rates, so using a low-boiling solvent increases the precipitation rate of the polymer in the solution, thereby promoting random precipitation of the polymer and formation of a porous structure, which is preferred.

The polymer concentration in the solvent may be 0.5 to 20 wt%.

On the stage of a spin coater, a laminated body of the metal catalyst substrate 15 and the graphene film 2 is fixed, and the prepared porous-membrane-forming solution is dropped onto the surface of the graphene film 2 near its center. The amount of the porous-membrane-forming solution dropped only needs to be sufficient to cover the graphene film 2. The rotation speed and rotation time of the spin coater are not particularly limited as long as a desired porous membrane can be formed. For example, the rotation speed may be 200 to 5000 rpm, and the rotation time may be 10 to 120 seconds. Thereafter, if the solvent remains, drying by heating is performed.

In preferred embodiments, the porous membrane 3 can be cast using an applicator or the like and formed on the two-dimensional layered material by a phase inversion method. The phase inversion method is a method of forming a porous structure when a material such as a polymer undergoes a phase transition from a liquid state to a solid state under controlled conditions. Typical phase inversion methods include nonsolvent-induced phase separation and thermally induced phase separation. In the nonsolvent-induced phase separation method, there is a phenomenon in which the polymer solution becomes unstable due to evaporation of the solvent in a polymer solution containing a nonsolvent or poor solvent, and the polymer precipitates in the presence of the nonsolvent; and a phenomenon in which, when the nonsolvent comes into contact with the polymer, the solvent in the polymer solution is extracted into the nonsolvent while the nonsolvent penetrates into the polymer solution, thereby destabilizing the polymer solution and causing the polymer to precipitate. The former is referred to as the dry phase separation method, and the latter as the wet phase separation method. A combination of both is referred to as the dry-wet phase separation method.

The method of forming a porous membrane by the phase inversion method is known, and reference may be made, for example, to Masahiro Tamura, Tadashi Uragami, and Mizuho Sugihara, "Permeation Characteristics on Cellulose Nitrate-Cellulose Acetate Blend Polymer Membranes," Journal of the Japan Society of Colour Material, Vol. 50 (6), 317-322 (1977). Since the nonsolvent-induced phase separation method forms a porous structure by utilizing the solvent exchange process between a good solvent and a poor solvent in a liquid phase, a porous membrane can be formed without causing excessive volume shrinkage due to rapid solvent evaporation or the like, thereby reducing damage to the graphene at the graphene/porous membrane interface during formation of the porous membrane.

The solvent used for the phase inversion method may be the same as that used for the spin coating method. The concentration of the polymer in the solvent may be 0.5 to 20 wt%.

After forming the porous membrane on the two-dimensional layered material in step (ii), step (iii) of removing the metal catalyst substrate by etching is carried out. The method for removing the metal catalyst substrate 15 may adopt a known technique. For example, when a copper foil is used as the metal catalyst substrate 15, etching can be performed using known solutions such as an aqueous ammonium persulfate solution or an aqueous iron(III) nitrate solution. The etching may be carried out by floating the laminated body on the etchant surface so that the copper foil surface is in contact with the etchant. Alternatively, the laminated body may be immersed in the etchant to etch the metal catalyst substrate. Since the laminated body of the present invention has high mechanical strength, even when immersed in an etchant, it can maintain a low sheet resistance.

When a two-dimensional layered material is also generated on the back surface of the metal catalyst substrate 15, where the porous membrane 3 is not formed, the two-dimensional layered material on the back surface is removed by oxygen plasma treatment or the like before etching the metal catalyst substrate 15. The porous membrane 3/two-dimensional layered material 2/metal catalyst substrate 15 is floated on the etchant so that the surface from which the two-dimensional layered material has been removed is in contact with the etchant, thereby removing the metal catalyst substrate 15. After removal of the metal catalyst substrate 15, the laminated body consisting of the porous membrane 3 and the two-dimensional layered material 2 is transferred to the surface of pure water in a water bath for washing. The washing step is preferably performed three times or more. The laminated body 1 floated on the pure water is scooped up with a membrane filter or the like. After scooping up, the laminated body 1 is dried, thereby completing production of the laminated body.

Hereinafter, the present invention will be described more specifically by way of Examples, but the invention is not limited to the following embodiments.

### (Example 1: Production of a laminated body comprising a graphene film and a porous membrane)

In this example, a laminated body composed of a monolayer graphene film and a cellulose membrane (porous membrane) (hereinafter also referred to as "porous membrane/graphene laminated body") was produced as follows.

Copper foil (35 µm thick) was prepared as the metal catalyst substrate for the growth of graphene by chemical vapor deposition (CVD). Prior to graphene film formation by CVD, the copper foil was sequentially immersed in pure water, ethanol, and acetone, followed by ultrasonic treatment to clean the surface of the copper foil. Thereafter, the copper foil was transferred into a thermal CVD furnace, hydrogen was introduced, and hydrogen annealing of the copper substrate was carried out at 1000 °C under a hydrogen atmosphere at a total pressure of 700 Pa for 30 minutes. Subsequently, methane was introduced, and CVD growth of graphene was carried out for 10 minutes at 1000 °C and a total pressure of 750 Pa with a flow rate of 2 sccm methane and 20 sccm hydrogen.

Using a spin coating method, a porous membrane was formed on the graphene film grown on the copper foil.

As the support layer for graphene transfer, nitrocellulose (CN), cellulose acetate (CA), or a mixed membrane of nitrocellulose and cellulose acetate (mixed cellulose ester membrane, MCE) was used.

For preparation of the porous-membrane-forming solution, nitrocellulose (Nacalai Tesque, product No. 24728-34), cellulose acetate (Sigma-Aldrich, product No. 419028), and acetone as a solvent were prepared. A solution was prepared by mixing cellulose acetate or nitrocellulose with acetone so that the content of cellulose acetate or nitrocellulose became 2 wt%. Further, three types of mixed cellulose ester solutions with different compositions were prepared by mixing nitrocellulose, cellulose acetate, and acetone so that the total content of nitrocellulose and cellulose acetate was 2 wt% and the weight ratios of nitrocellulose to cellulose acetate were 3:1, 1:1, and 1:3. As a comparative sample, a PMMA solution, which has been conventionally used as a transfer support film for graphene transfer, was prepared. PMMA (Sigma-Aldrich, product No. 182265) was dissolved in ethyl lactate as a solvent to prepare a PMMA solution with a PMMA content of 4 wt%.

Next, the porous-membrane-forming solution was dropped onto the graphene film grown on the copper foil, and spin coating was carried out at 2000 rpm to form a nitrocellulose membrane, a cellulose acetate membrane, or a mixed cellulose ester membrane. Similarly, under the same conditions, a PMMA film as a transfer support film was formed on the graphene film grown on the copper foil. After formation of the porous membrane or the transfer support film, the unnecessary graphene film formed on the back side of the copper foil was removed by oxygen plasma treatment. Thus, a laminated body consisting of a porous membrane/graphene film/copper foil was obtained. As a comparative example, a laminated body composed of a PMMA film (transfer support film)/graphene film/copper foil was obtained.

An etching bath was prepared, and was filled with an aqueous solution of iron(III) nitrate (0.5 mol/L) at 25 °C as a copper etchant. The size of the laminated body composed of a porous membrane/graphene film/copper foil to be subjected to copper etching was 15 mm × 10 mm. The laminated body was floated on the copper etchant with the copper foil side in contact with the solution, and the copper foil of the laminated body was removed by etching for 5 hours. Thus, a porous membrane/graphene laminated body composed of a porous membrane and a graphene film was obtained. A laminated body composed of a PMMA film (transfer support film)/graphene film was also obtained as a comparative sample.

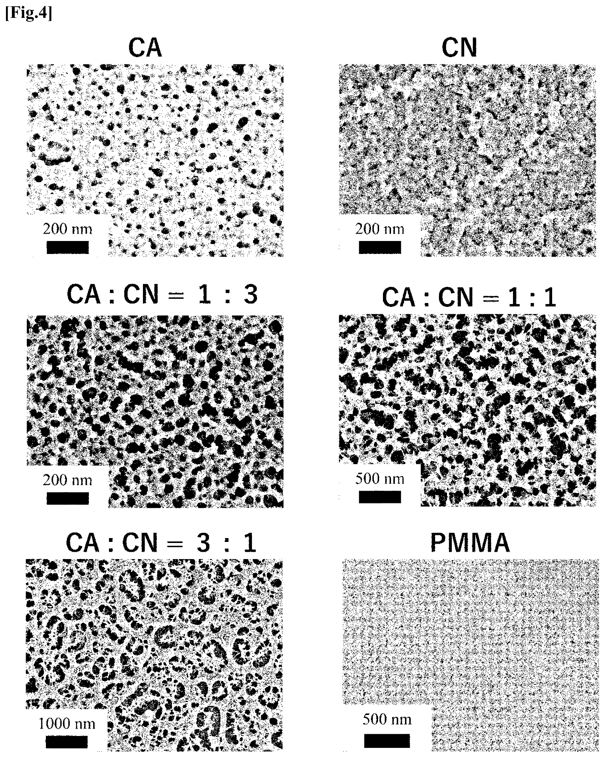

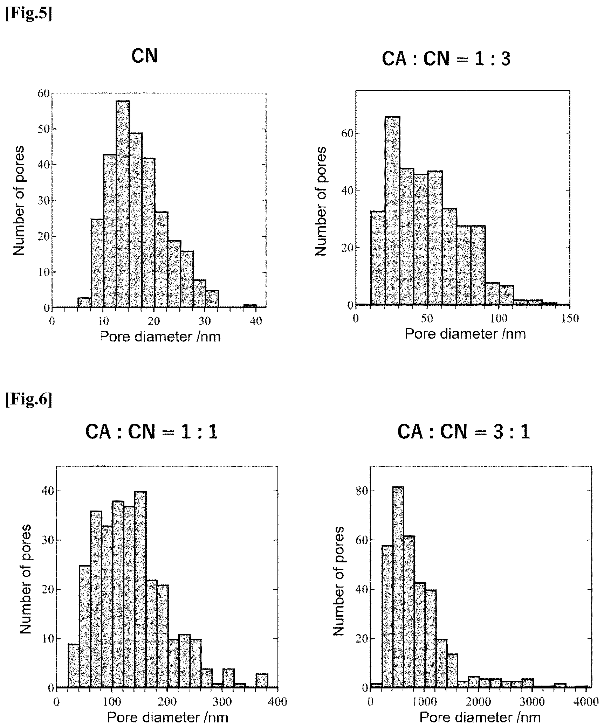

The surface of the porous membrane of the fabricated porous membrane/graphene laminated body was observed using FE-SEM (ZEISS), and the pore diameter and the pore area ratio were measured from the SEM images obtained. The samples used for SEM observation were prepared by coating the surface of the porous membrane of the porous membrane/graphene laminated body formed on the copper foil after spin coating with a 3 nm platinum layer. Similarly, the surface of the PMMA film used in the comparative example was also observed. The SEM images are shown in Fig. 4. The PMMA film had a smooth surface and formed a dense film structure without pores. The CN membrane and the CA membrane had pores, but they consisted of small pores, and no pores larger than 100 nm were observed. The CA membrane had a surface with a low pore density. All the fabricated mixed cellulose ester membranes had porous structures, and it was observed that the higher the weight ratio of nitrocellulose, the larger the pore diameter tended to be. The diameters of all pores recognizable in the SEM images were estimated based on the scale bar. From these pore diameter data, the pore area ratio (the area fraction of pores relative to the surface of the porous membrane) was calculated for the CN membrane. For the mixed cellulose ester membrane, the SEM images were converted to black-and-white (binary) images, and the black area fraction was calculated as the pore area ratio. The pore size distributions (histograms) of each sample are shown in Figs. 5 and 6. Accordingly, histograms are shown for all samples except the PMMA film (no pore) and the CA membrane (low pore density). The CN membrane had only pores with diameters of 40 nm or less, with an average pore diameter of 17 nm. The pore area ratio of the CN membrane was 2.1%. The mixed cellulose ester membrane with CN:CA = 1:3 had only pores of 150 nm or less, with an average pore diameter of 49 nm. The mixed cellulose ester membranes with CN:CA= 1:1 and CN:CA = 3:1 had pores larger than 150 nm, with average pore diameters of 140 nm and 880 nm, respectively. The CN:CA = 3:1 membrane had many large pores of around 550 nm in diameter, as well as a pore size distribution including even larger pores of 1.5 µm or more. The pore area ratio of the mixed cellulose ester membranes with CN:CA = 1:3, CN:CA = 1:1, and CN:CA = 3:1 was 38%, 40%, and 32%, respectively.

### (Example 2: Production of a laminated body comprising a porous membrane, a graphene film, and a transparent insulating substrate)

In this example, the porous membrane/graphene laminated body produced in Example 1 was transferred onto a transparent insulating substrate to produce a laminated body comprising a porous membrane, a graphene film, and a transparent insulating substrate. As the transparent insulating substrate, a quartz glass substrate (thickness: 1 mm, 20 mm × 20 mm) was prepared. On part of the quartz glass substrate, 10 nm of chromium was deposited using a vacuum deposition apparatus, and 50 nm of gold was further deposited on top of the chromium. This metal-deposited film functions as a contact electrode when the porous membrane/graphene laminated body is laminated onto the quartz glass substrate, and is necessary when the transferred graphene film is applied as a device such as an electrode for electrochemical measurements.

A water bath filled with ultrapure water was prepared. After copper etching, the porous membrane/graphene laminated body floating in the etchant was transferred onto the surface of the water bath and washed. The laminated body was further transferred into another water bath to repeat the washing process. This washing step was performed three times. The porous membrane/graphene laminated body floating in the ultrapure water bath was scooped up using a quartz glass substrate that had been pre-deposited with chromium and gold to serve as a contact electrode. In this process, the porous membrane/graphene laminated body was laminated so that a portion of it overlapped with part of the contact electrode. In this manner, a laminated body comprising a quartz glass substrate as the transparent insulating substrate, a porous membrane, and a graphene film was produced.

The thickness of the porous membrane on the produced laminated body was measured using a stylus profilometer (Bruker Dektak). The thickness of the laminated body on a quartz glass substrate produced using a 2 wt% cellulose acetate and nitrocellulose mixed solution (CN:CA = 3:1) was about 400 nm. In contrast, the thickness of the laminated body produced using an ethyl lactate solution containing 4 wt% PMMA was about 150 nm.

### (Example 3: Preparation of a self-supporting graphene/porous membrane laminated body)

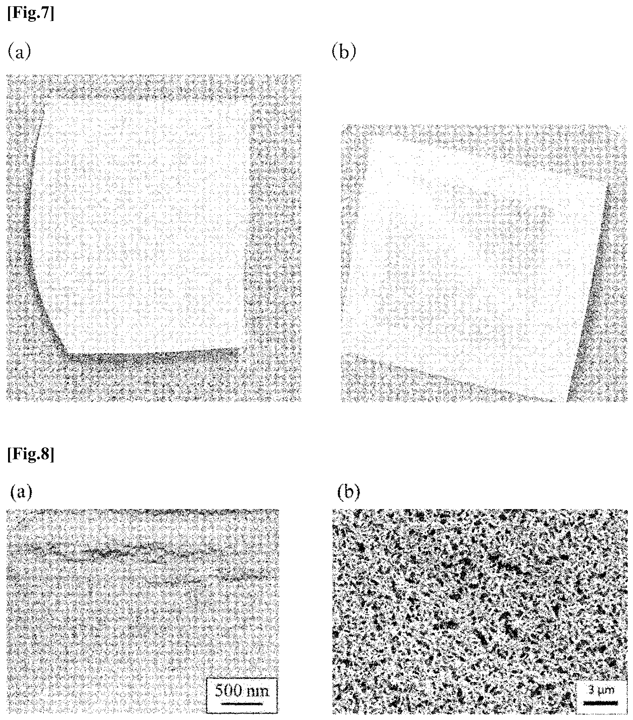

A PMMA/graphene laminated body and a mixed cellulose ester/graphene laminated body (CN:CA = 3:1), both produced under the same conditions as in Example 1, were transferred from pure water onto a 47 mm-diameter membrane filter (ADVANTEC, model A045A047A) so that the transfer support materials of the respective laminated bodies were in contact with the membrane filter, and then dried. As a result, the PMMA/graphene laminated body became wrinkled (Fig. 7(a)), whereas the mixed cellulose ester/graphene laminated body maintained planarity (Fig. 7(b)). This difference between the two transfer support materials can be regarded as reflecting the fact that the porous structure relieves the internal stress of the laminated body. However, when attempting a transfer method without floating on water (Fig. 2A) using this laminated body, the laminated body tore during the process of bringing the graphene into contact and peeling, and could not be transferred onto the substrate. This is considered to be because, compared with the thicker porous membranes described in later Examples, the porous membrane prepared by the spin coating method adhered more strongly to the membrane filter. Since a thinner porous membrane has flexibility to follow fine irregularities, the adhesion area with the membrane filter increases, resulting in stronger adhesion.

### (Example 4: Verification of the transfer support capability of porous membranes)

In this example, the transfer support ability of the porous membrane during the etching step of the metal catalyst substrate or the subsequent transfer step to a transparent insulating substrate was verified.

Except that an acetone solution of nitrocellulose at a concentration of 1 wt% was used, a porous membrane was formed on a graphene film under the same conditions as in Example 1. The thickness of the porous membrane formed on the graphene film was less than 100 nm. When copper-foil etching was performed after porous membrane formation, tearing of the membrane occurred due to insufficient supporting strength.

Similarly, porous membranes were formed on graphene films under the same conditions as in Example 1, except that acetone solutions containing 6 wt% nitrocellulose or 4 wt% cellulose acetate were used. The porous membranes formed on the graphene films had thicknesses of 1 µm or more. Although the porous membranes were able to support the graphene film during the copper foil etching step, partial delamination from the quartz substrate was observed after transfer. On the other hand, when a PET substrate was used instead of a quartz glass substrate, no delamination occurred even when the porous membrane thickness was 1 µm or more.

### (Example 5: Investigation of the fabrication method of a porous membrane/graphene laminated body by solution casting)

In this example, a mixed cellulose ester membrane was formed using a solution casting method with an applicator, and methods for rendering the mixed cellulose ester membrane porous were examined. The mixed cellulose ester membrane used in this example was prepared at a weight ratio of CA:CN = 1:2. First, solvents were examined. A porous-membrane-forming solution was prepared by dissolving 12 wt% mixed cellulose ester in two types of solvents: acetone, and a mixed solvent of acetone and formamide (weight ratio 1:1). The solution was cast manually onto a copper substrate using an applicator with a gap height of 75 µm (BEVS four-sided applicator, width 60 mm, model BEVS 1803/60/F13). Fig. 8 shows SEM images of the surface of the mixed cellulose ester membrane after drying following solution casting (platinum-coated to suppress charging). When acetone was used as the solvent, no pore structure was observed, whereas when the acetone/formamide mixed solvent was used, a pore structure was observed. Acetone is a good solvent for both CA and CN, whereas formamide is a good solvent only for CN and a poor solvent for CA. In the acetone/formamide mixed solvent, as the good solvent acetone evaporates during casting, the solvent quality of the solution gradually becomes poorer, and phase separation occurs. It is considered that porosity was introduced in the acetone/formamide mixed solvent by this phase separation. This method is a dry phase inversion method.

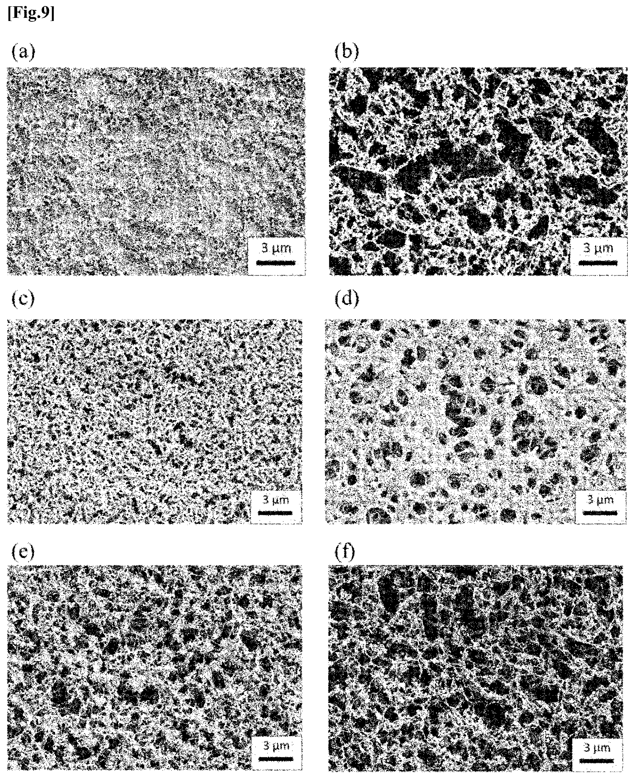

Next, a dry-wet phase inversion method was examined, in which the cast film was immersed in ultrapure water after solution casting. At the same time, the effect of solute concentration of the mixed cellulose ester solution on the porous structure was also evaluated. Mixed cellulose ester-forming solutions were prepared by dissolving CN and CA in an acetone/formamide mixed solvent (weight ratio 1:1) at concentrations of 4, 8, and 12 wt%. The solutions were cast manually onto copper substrates using an applicator with a gap height of 75 µm. For each solute concentration, two types of samples were prepared: a sample dried after casting (dry process), and a sample immersed in ultrapure water for 24 hours after 60 seconds from casting and then dried (dry-wet process). The surfaces of the fabricated mixed cellulose ester membranes were platinum-coated, and their surface structures were observed by SEM. Fig. 9 shows SEM images of the surfaces of six mixed cellulose ester membranes prepared: (a) 4 wt% without immersion, (b) 4 wt% with immersion, (c) 8 wt% without immersion, (d) 8 wt% with immersion, (e) 12 wt% without immersion, and (f) 12 wt% with immersion. In all solute concentrations, it was observed that immersion in water resulted in an increase in pore diameter. This is because immersion in water caused gelation of the casting solution, resulting in replacement of the solvent of the casting solution with water, and phase separation occurred. Table 1 shows the thickness of the porous membranes measured by a stylus profilometer (Bruker Dektak).

From the film thickness measurement results, it was observed that both increasing solute concentration and immersion in water caused an increase in film thickness. Since immersion in water significantly increased the thickness of the porous membrane, it indicates that the immersed samples contained many voids. With increasing solute concentration, the amount of solute contained in the casting solution increased, which also resulted in increased film thickness. In the 12 wt% sample immersed in water, the remarkable increase in film thickness is considered to be related to the viscosity of the porous-membrane-forming solution. As the viscosity increased, the fluidity of the solute decreased and aggregation of the solute was suppressed, thereby reducing shrinkage of the porous membrane thickness relative to the applicator gap. Table 2 shows the viscosity of the porous-membrane-forming solutions measured with a viscometer (Anton Paar, model ViscoQC 100, spindle SC4-21).

**[Table 1]**

| Solute concentration | Water immersion | Film thickness (µm) |
|---|---|---|
| 4 wt% | No | 1.0 |
| 4 wt% | Yes | 2.7 |
| 8 wt% | No | 1.3 |
| 8 wt% | Yes | 5.5 |
| 12 wt% | No | 3.5 |
| 12 wt% | Yes | 16 |

**[Table 2]**

| Solute concentration | Speed /rpm | Viscosity /mPa·s | Torque /% |
|---|---|---|---|
| 4 wt% | 50 | 37 | 23.1 |
| 8 wt% | 50 | 138 | 86.4 |
| 12 wt% | 12 | 609 | 91.4 |

Next, whether graphene grown on a copper foil could be transferred onto a quartz glass substrate using these mixed cellulose ester membranes was examined. Using mixed cellulose ester membranes prepared with an acetone solvent solution, as in the sample of Fig. 8(a), transfer of graphene to a quartz glass substrate was attempted according to the same procedure as in Example 2. As a result, these laminated bodies delaminated from the quartz glass substrate during the drying process after transfer and could not be transferred onto the quartz glass substrate. On the other hand, samples prepared using porous-membrane-forming solutions with a formamide/acetone mixed solvent were successfully transferred onto the quartz glass substrate for all laminated bodies produced under the conditions of Table 1, without delamination even after drying. Since the samples prepared with an applicator had increased thickness compared with those prepared by the spin coating method, it is considered that tensile stress also increased. In the case of mixed cellulose ester membranes prepared from an acetone solvent solution, no porous structure was formed, whereas in the acetone/formamide mixed solvent, a porous structure was formed throughout the membrane, which is considered to have relieved the tensile stress and prevented delamination. Thus, it was found that by making the support film porous, even when the thickness was 10 µm or more, the support film/graphene laminated body could be transferred onto a substrate without delamination.

Fig. 10(a) shows the Raman spectrum of a porous mixed cellulose ester/graphene laminated body transferred onto a quartz glass substrate using a support film prepared from a 12 wt% solution and then immersed in pure water. Peaks attributable to CH₃ and C-H of cellulose, appearing around 1280 cm⁻¹ and 2950 cm⁻¹, were observed; however, the 2D peak and G peak attributable to graphene were not observed. This is considered to be because the support film was porous and thick, and thus the laser light was scattered by the support film and did not reach the graphene. Subsequently, Raman spectroscopy was carried out after immersing the mixed cellulose ester/graphene laminated body in heated acetone for 20 minutes to remove the mixed cellulose ester membrane serving as the support film. Fig. 10(b) shows the Raman spectrum of the graphene after removal of the support film. The 2D peak and G peak attributable to graphene were observed, and the D peak around 1350 cm⁻¹, which indicates defects, was hardly observed, indicating that high-quality graphene could be produced even when a mixed cellulose ester membrane prepared by the solution casting method was used as a support material. Furthermore, since the peak attributable to the C-H of cellulose appearing around 2950 cm⁻¹ was not observed, it was confirmed that the mixed cellulose ester was completely removed by immersion in heated acetone.

### (Example 6: Change in thickness of mixed cellulose ester membranes prepared by the phase inversion method (1))

To evaluate the effect of porous membrane thickness, a 12 wt% acetone/formamide mixed solution (acetone:formamide = 1:1 by weight) of a mixed cellulose ester with a cellulose acetate:nitrocellulose weight ratio of 2:1 was used to prepare porous membranes under conditions with applicator gap heights of 25, 50, 75, and 100 µm using a four-sided applicator (width: 60 mm). For casting of the cellulose solution with the applicator, a fully automatic linear motion coater, the ALC-mini2 (COTEC), capable of controlling the coating speed was used. Graphene was grown on copper foil in the same manner as in Example 1. In this experiment, the coating speed during membrane formation was set at 2 mm/s, and four samples with different thicknesses of porous membranes were formed on the graphene grown on copper foil. The solution had high viscosity (609 mPa·s). Because drying started immediately after the solution was spread on the graphene, the solution did not spread further after being applied with the applicator. After coating the copper foil with the cellulose solution, the coated copper foil was immersed in a pure water bath for 1 hour, removed from the pure water, and then dried naturally.

To analyze the surface structure of the fabricated porous mixed cellulose ester/graphene laminated bodies, SEM observation of the membrane surfaces was carried out in the same manner as in Example 1, and the average pore diameter and pore area ratio were calculated from the obtained images. In addition, to investigate how the actual membrane thickness changed depending on the applicator gap height, the membrane thicknesses were measured using a laser microscope. Fig. 11 shows SEM images of the mixed cellulose ester membranes. Comparison of the samples revealed that as the membrane thickness increased, the cellulose fiber structure became thicker. Table 3 shows the membrane thicknesses, average pore diameters of the pores on the membrane surface, and pore area ratios.

Comparison of the four samples showed that the membrane thickness increased with the applicator gap height. In addition, as the membrane thickness increased, the average pore diameter decreased, and the pore area ratio also decreased. This is considered to be because when the cellulose solution was applied with an applicator and immersed in water, solvent exchange was less likely to occur in the thicker membranes, and voids were less likely to form upon drying. When these four laminated bodies were transferred onto quartz glass substrates according to the procedure of Example 2, none of the laminated bodies exhibited delamination after drying.

**[Table 3]**

| Gap height /nm | Film thickness /µm | Surface average pore diameter /nm | Pore area ratio /% |
|---|---|---|---|
| 25 | 2.6 | 270 | 25.9 |
| 50 | 6.5 | 271 | 23.7 |
| 75 | 11.0 | 253 | 21.1 |
| 100 | 18.5 | 213 | 11.3 |

### (Example 7: Change in thickness of mixed cellulose ester membranes prepared by the phase inversion method (2))

To investigate the effect of membrane thickness when forming thicker porous membranes, a mixed cellulose ester solution (12 wt%, CA:CN = 1:2 by weight) in an acetone/formamide mixed solvent (acetone:formamide = 1:1 by weight) was used, and porous membranes were prepared by the phase inversion method under conditions with applicator gap heights of 100, 200, 300, and 400 µm, using a multi-gap applicator (COTEC, width 100 mm, model MA100). First, porous membranes were prepared on copper foil without graphene growth. Except for the type of applicator, the gap height, and the coating speed, the coating conditions were the same as in Example 6. In this example, the coating speed of the applicator was set to 15 mm/s. A photograph of the sample coated at a gap height of 300 µm, after immersion in pure water and drying, is shown in Fig. 12. The large warpage observed in the copper foil substrate suggests that the tensile stress within the porous membrane increased as the membrane thickness increased. For samples with gap heights of 200 µm or more, warpage of the copper foil and partial delamination of the porous membrane were observed. For the sample with a gap height of 100 µm, almost no warpage of the copper foil was observed. Portions (35 mm × 35 mm) without delamination were cut from the porous membranes (100 mm × 100 mm), and the copper foil was etched by floating the cut samples on a 10 wt% aqueous ammonium persulfate solution. The porous membranes were then transferred to glass slides (75 mm × 52 mm, Matsunami Glass, product No. S9111). The state after transfer is shown in Fig. 13. After transfer to the glass slide, washing with pure water and drying were carried out. While the porous membranes adhered to the glass slides maintaining planarity when wet, the samples with gap heights of 200 µm or more delaminated from the glass slides during drying. Only the sample prepared with a gap height of 100 µm remained adhered to the glass slide even after drying. The sample with a gap height of 200 µm exhibited delamination over more than 50% of its area, while the samples with gap heights of 300 µm or more exhibited almost complete delamination. The membrane thicknesses measured with a laser microscope were 37 µm for the sample prepared with a gap height of 100 µm, 75 µm for the 200 µm sample, and 157 µm for the 300 µm sample.

Next, mixed cellulose ester membranes (12 wt%, CA:CN = 1:2) were prepared on copper foils (35 mm × 70 mm) with monolayer graphene grown thereon under the same four gap height conditions. Fig. 14 shows photographs of the porous membranes after immersion in water and drying. It was observed that warpage of the copper foil increased with increasing gap height. Since cracks occurred in the 400 µm sample, three types of samples with gap heights of 100, 200, and 300 µm were cut into 20 mm × 20 mm pieces and transferred onto glass slides. After washing with pure water and drying, no delamination of the laminated bodies from the glass slides was observed. Despite the large stress generated, as shown in the photographs of Fig. 14, no delamination from the glass slides occurred, indicating that the adhesion at the graphene/glass slide interface and the graphene/porous membrane interface was strong. However, in the laminated body prepared with a gap height of 300 µm, cracks occurred in the sample during the fabrication step before etching of the copper, and partial tearing was observed in the laminated body after transfer. Thus, it was found that increasing the membrane thickness increases the tensile stress of the porous membrane, which is likely to degrade the quality of graphene in the laminated body. As also shown in Example 6, increasing the membrane thickness tends to form a layer of smaller pore sizes, known as a skin layer, on the porous membrane surface. This skin layer is considered to reduce the stress-relaxation function of the porous membrane. On the other hand, the smoothness and flexibility of graphene enhance adhesion to the substrate and improve adhesion by van der Waals forces. These results also indicate that porous mixed cellulose ester membranes with a thickness of 100 µm or less have high adhesion to graphene and high resistance to deformation, and possess sufficient mechanical strength as a transfer support material for graphene.

### (Example 8: Examination of polymer materials suitable for porous membranes for graphene transfer support)

Six types of polymers were cast onto graphene grown on copper foil by a CVD method, and porosification by a phase inversion method was attempted. To confirm whether the fabricated polymer membranes formed a porous structure, SEM observations were carried out on the polymer membrane/graphene laminated bodies. The polymers used for the polymer membranes were cellulose acetate (CA), nitrocellulose (CN), mixed cellulose ester (MCE), polymethyl methacrylate (PMMA), polyvinylidene fluoride (PVDF), and polyethersulfone (PES).

Copper foil was cut into 110 mm × 70 mm pieces, immersed in acetic acid for 3 minutes, then sequentially immersed in pure water and acetone, and then dried under a nitrogen stream. Thereafter, the copper foil was transferred into a thermal CVD furnace, hydrogen was introduced, and hydrogen annealing of the copper substrate was performed under conditions of a hydrogen atmosphere at a total pressure of 700 Pa and a temperature of 1000 °C for 30 minutes. Then, methane was introduced, and graphene CVD growth was carried out for 10 minutes at 1000 °C and a total pressure of 750 Pa with a flow rate of 2 sccm methane and 20 sccm hydrogen. The copper foil on which graphene had been grown was then cut into 110 mm × 35 mm pieces. This CVD process was repeated three times to prepare six copper foils with graphene grown thereon, each of 110 mm × 35 mm.

The compositions of the solutions for preparing the respective polymer membranes are shown in Table 4.

**[Table 4]**

| Material | Solute product code | Solute concentration | Solvent composition | |
|---|---|---|---|---|
| CA | Aldrich 419028 | 12 wt% | Acetone: | 44 wt% |
| | | | DMSO: | 44 wt% |
| CN | Nacalai Tesque 24728-34 | 12 wt% | Acetone: | 42 wt% |
| | | | Formamide: | 42 wt% |
| | | | IPA: | 4 wt% |
| MCE | Mixed of the above two | 12 wt% (CA 4 wt%, CN 8 wt%) | Acetone: | 43 wt% |
| | | | Formamide: | 43 wt% |
| | | | IPA: | 2 wt% |
| PMMA | Aldrich 182265 | 12 wt% | Acetone: | 44 wt% |
| | | | DMF: | 44 wt% |
| PVDF | Aldrich 347078 | 12 wt% | Acetone: | 44 wt% |
| | | | DMF: | 44 wt% |
| PSE | Aldrich 191094 | 16 wt% | NMP: | 82 wt% |
| | | | IPA: | 2 wt% |

The polymer materials listed in Table 4 were weighed into vials, and solvents were added and the mixtures were stirred. Since PVDF and PES were difficult to dissolve, the solutions were maintained at 55 °C until completely dissolved. The copper foil (110 mm × 35 mm) with graphene grown thereon was fixed on the stage using the clamp function of an automatic linear motion coater, and the solutions were cast onto the graphene/copper foil using a multi-gap applicator. The micrometer reading of the applicator gap height was set to 160 µm. Considering that the copper foil thickness was 35 µm, the actual gap height between the copper foil and the blade was estimated to be about 125 µm. The applicator speed was set to 15 mm/s. The graphene/copper foils coated with each solution were immediately immersed in a pure water bath and removed after 30 minutes. After removal from the pure water bath, polymers adhering to the graphene on the back side of the copper foil were wiped off using cotton swabs wetted with appropriate solvents, and the graphene on the back side of the copper foil was removed by exposure to oxygen plasma. The foils were then cut into 15 mm × 15 mm pieces.

Photographs of the six types of samples are shown in Fig. 15. The four samples coated with PVDF, PES, CN, or MCE exhibited a whitish appearance, suggesting the formation of porous structures. On the other hand, the samples coated with CA and PMMA showed the color of the copper foil clearly, indicating that the polymer support films were almost transparent and that porous structures had not been formed. The polymer-film side of the polymer film/graphene/copper foil laminated bodies was coated with platinum, and SEM observation was conducted. SEM images are shown in Figs. 16 and 17. Pores were observed in all polymer films, but the surface pores of CA, PMMA, and PES were small, and the pore area ratio was low. In the case of PES, since the sample appeared whitish, it is considered that a skin layer had formed on the surface. For the other three polymers (CN, MCE, and PVDF) as well, the small pore area ratio at the surface suggests that the thicker films resulting from the large applicator gap height caused the formation of skin layers.

### (Example 9: Transfer of laminated bodies by a conventional method and measurement of film thickness)

The 15 mm × 15 mm polymer support film/graphene/copper foil laminated bodies prepared in Example 8 were floated on a 10 wt% ammonium persulfate aqueous solution, used as the copper etchant, with the copper side facing downward. After the copper was completely etched away, the polymer support film/graphene laminated bodies floating on the etchant were transferred to the surface of a water bath and washed, as in Example 2. The laminated bodies were further moved to separate water baths to repeat the washing process. This washing step was repeated three times. The polymer support film/graphene laminated bodies floating on the pure water bath were scooped up using quartz glass substrates. The polymer support film/graphene/quartz glass substrate laminated bodies thus obtained were naturally dried. A photograph of the polymer support film/graphene laminated bodies transferred onto quartz glass substrates is shown in Fig. 18. The CA/graphene laminated body delaminated from the quartz glass substrate during the drying process. From the partially translucent regions seen in the CA photograph, it is suggested that partial porous structures were formed, but since most of the film was transparent and sufficient porous structure had not been formed, tensile stress developed, exceeding the adhesion between the quartz glass substrate and graphene. The PMMA/graphene laminated body, compared to the other four support films/graphene laminated bodies except for CA, was more transparent, yet the entire film appeared uniformly translucent.

The thicknesses of the support film/graphene laminated bodies transferred onto quartz glass substrates were measured using a laser scanning confocal microscope (Keyence, VK-X1000). The thicknesses of the five laminated bodies are shown in Table 5. Since the CA film could not be transferred onto the substrate, its thickness could not be measured. Even with the same applicator gap height, differences in thickness were observed among the samples, which are considered to be related to solute concentration, solution viscosity, and the degree of porous structure formation.

**[Table 5]**

| Sample | Film thickness (µm) |
|---|---|
| CN | 18.0 |
| MCE | 28.8 |
| PMMA | 3.2 |
| PVDF | 17.3 |
| PES | 47.0 |

### (Example 10: Fabrication of graphene/porous support film free-standing laminated bodies and structural evaluation by SEM)

The 15 mm × 15 mm polymer support film/graphene/copper foil laminated bodies prepared in Example 8 were floated on a 10 wt% ammonium persulfate aqueous solution, used as the copper etchant, with the copper side facing downward, in the same manner as in Example 8. After complete etching of the copper, the graphene/polymer support film laminated bodies floating in the aqueous solution, as shown in Fig. 19, were scooped up with a mixed cellulose ester membrane filter (ADVANTEC, product No. A045A047A) such that the support film was in contact with the membrane filter. The membrane filter portion of the laminated body was held with tweezers, and the membrane filter carrying the polymer film/graphene laminated body was immersed in a pure water bath to wash the polymer film/graphene laminated body. At this time, the laminated body detached from the membrane, with the porous membrane side contacting the water surface, and could be floated on the water surface with the graphene side facing upward. After washing in the water bath, the laminated body was again scooped up so that the membrane filter contacted the support film. The laminated body floating on the water bath could be easily transferred onto the membrane filter. The laminated body removed from the water bath could remain free-standing without bending even when detached from the membrane filter, but handling was easier when using the membrane filter since contact with the graphene side of the laminated body could be avoided. A photograph of the graphene/polymer support film laminated body placed on the membrane is shown in Fig. 20. The laminated body supported with PMMA showed some thin whitish regions, but was mostly transparent. The graphene surfaces of five types of graphene/polymer support film laminated bodies were observed by SEM. SEM images are shown in Fig. 21. Although the polymer materials used as transfer support films were all electrically insulating, charging could be avoided because graphene was transferred onto the top surface. Moreover, since graphene is a single-atomic-layer sheet, electron beams could penetrate it, allowing the structure of the polymer support films to be observed. Even for CN, MCE, PVDF, and PES, which appeared whitish to the naked eye, the SEM images of the polymer-side surfaces (Fig. 16) showed the formation of skin layers with low pore area ratios. However, the structures of these four polymers near the graphene/polymer interface had sufficiently porous structures. CN, MCE, and PVDF exhibited mesh-like networks, whereas PES exhibited fragmented network-like structures rather than networks. For PES, the charging effect during observation was stronger than for the other four materials, making observation difficult, suggesting significant graphene damage and reduced electrical conductivity. The SEM image of the graphene/PMMA laminated body showed low contrast and a wavy structure, appearing as though PMMA induced wrinkles by gripping the graphene film. In general, when polymer films are formed by solution casting, shrinkage during solvent evaporation causes residual stress in the polymer. In this study, although the polymer films were solidified by solvent exchange rather than solvent evaporation, the process can be regarded as causing similar volumetric shrinkage. When such residual stress occurs, it exerts a compressive force on the adhered graphene. As the polymer thickness increases, the stress resulting from volumetric shrinkage also increases, thereby applying a greater compressive force to the graphene. The absence of wrinkle-like structures observed at the graphene/support film interface in the SEM images of CN, MCE, PVDF, and PES is considered to be because porous structures formed near the graphene/support film interface avoided excessive volumetric shrinkage and relieved the stress. In contrast, the PMMA support film likely did not form a sufficiently porous structure to mitigate the stress.

During SEM observation, if the graphene layer of a sample were torn and the underlying polymer film exposed, localized charging would be expected to become more pronounced in those regions; however, such areas were not observed in any of the samples.

### (Example 11: Transfer of graphene/porous support film free-standing laminated bodies and four-terminal resistance measurement)

The graphene/porous membrane free-standing laminated bodies fabricated in Example 10 were transferred onto quartz glass substrates with Au/Cr deposited using the method shown in Fig. 22. As illustrated in Fig. 20, a graphene/porous support film free-standing laminated body was placed on a membrane filter (corresponding to the state shown in Fig. 22(1)). The underlying membrane filter beneath the porous support film was wetted with pure water (Fig. 22(2) and (3)). Pure water was then dropped onto the graphene film of the laminated body (Fig. 22(4)), and a 20 mm × 20 mm quartz glass substrate with Au/Cr (5 mm × 5 mm) deposited on its four corners was placed on top (Fig. 22(5) and (6)). After excess water was absorbed with a nonwoven fabric such as a cellulose-based fabric (e.g. BENCOT, a trade name of Asahi Kasei Corporation) and moderately dried, additional water was applied from the membrane filter side, allowing the porous support film to be easily separated from the membrane filter. After drying at room temperature, a porous membrane/graphene laminated body transferred onto the desired position was obtained, as shown in Fig. 22(7).

In general CVD graphene transfer processes, as in Example 2, there is a step in which the support film/graphene laminated body floating on the surface of a pure water bath is scooped up. In contrast, the present method, by preparing a graphene/porous membrane free-standing laminated body, enables the facile transfer of graphene onto arbitrary substrates at arbitrary positions without the need to float the laminated body on water.

The electrical properties of graphene fabricated using five types of graphene/porous support film free-standing laminated bodies were evaluated by means of van der Pauw resistivity measurements and Hall-effect measurements, using a measurement system manufactured by Nanometrics. After the electrical characterization, the samples were immersed in acetone to attempt dissolution of the support films. CN, MCE, and PMMA could be removed, whereas PES and PVDF could not be completely dissolved. For the samples after dissolution of the support films, Hall-effect measurements and Raman spectroscopy were also carried out.

Subsequently, PES could be removed by immersion in NMP, which had been used as the solvent for the porous film-forming solution. PVDF was immersed in heated DMF for 30 minutes, but complete dissolution was not achieved. The electrical properties and micro-Raman spectroscopy of these samples were again measured. The Raman spectra are shown in Fig. 23, and the optical microscope images during Raman spectroscopy are shown in Fig. 24. Table 6 shows the electrical properties of the five types of samples before and after dissolution of the support films. For reference, the sheet resistance of graphene fabricated by the conventional PMMA transfer method was about 550 Ω/sq.

**[Table 6]**

| Sample (support film) | Condition | Sheet resistance [Ω/sq] | Mobility [cm²/Vs] | Carrier density [10¹³ cm⁻²] |
|---|---|---|---|---|
| CN1 | With support film | 164.3 | 767 | 4.95 |
| | After acetone immersion | 178.2 | 715 | 4.90 |
| CN2 | With support film | 172.8 | 768 | 4.70 |
| MCE1 | With support film | 216.6 | 796 | 3.62 |
| | After acetone immersion | 259.4 | 1040 | 2.31 |
| MCE2 | With support film | 171.9 | 860 | 4.22 |
| PMMA | With support film | 3997 | 80.8 | 1.93 |
| | After acetone immersion | Not measurable | | |
| PVDF | With support film | 802.8 | 139 | 5.60 |
| | After acetone immersion | 7140 | 14.5 | 6.02 |
| PES | With support film | 2198 | 51.1 | 5.56 |
| | After acetone immersion | Not measurable | | |

In the Raman spectra in Fig. 23, the samples fabricated using CN, MCE, and PMMA as support films, after acetone dissolution, exhibited spectra characteristic of monolayer graphene. In contrast, the samples with PES and PVDF support films contained significant residues, as shown in Fig. 24, and exhibited peaks other than those of graphene in their Raman spectra. For the PVDF-supported transfer sample, residue-derived peaks were dominant, with only a slight 2D peak attributable to graphene observed. The sheet resistance of the PVDF-supported transfer sample markedly increased after acetone immersion; however, because measurable values were still obtained, this indicates that graphene remained beneath the polymer residue even after acetone immersion. In the optical microscope images in Fig. 24, numerous tears in the graphene were observed after immersion in DMF, and electrical properties could not be measured. For the PES-supported transfer sample, the sheet resistance before dissolution of the support film was about ten times higher than that of the MCE- and CN-supported transfer films, and after acetone immersion, the electrical properties could not be measured. As shown in the optical microscope images in Fig. 24, the PES residues appeared to cover almost the entire sample. After immersion in NMP, PES could be dissolved; however, most of the graphene on the substrate was torn or delaminated. PES-supported transfer was also performed using the conventional scooping method for transfer onto a substrate for resistivity measurement. The sheet resistance of this sample was 791 Ω/sq, suggesting that numerous tears occurred in the process of lifting the laminated body from the water surface. As indicated by the SEM images in Fig. 21, the PES structure at the graphene/support film interface, unlike those of CN, MCE, and PVDF, did not form a mesh-like network; accordingly, it is considered that the ability to support the graphene during lifting from the water surface was insufficient. These results indicate that differences in how the porous structure of the support material is formed also affect the quality of the transferred graphene.

Graphene transferred using CN and MCE as support films exhibited almost no tears even after acetone immersion and maintained high electrical conductivity. For both samples, sheet resistances below 200 Ω/sq were obtained, which is an extremely low value for monolayer graphene. This low sheet resistance indicates that high-quality, tear-free graphene was successfully transferred. In contrast, the PMMA-supported transfer sample exhibited visible residues after acetone immersion, which were not removed even after immersion in hot acetone. Optical microscope images after acetone immersion revealed that most of the graphene had torn. Based on the SEM image in Fig. 21, it is considered that a thick film with insufficient porosity induced large tensile stress, resulting in numerous tears. The residues that were insoluble in hot acetone are considered to be PMMA trapped within the torn graphene and remaining on the substrate surface.

From these results, it can be concluded that MCE or CN porous membranes, which, owing to their porous structure, do not induce significant stress and can be readily dissolved after graphene transfer, are the optimal support films for transferring graphene. Furthermore, since the graphene/porous membrane laminated body can be self-supporting, it was demonstrated that high-quality graphene can be transferred by a simple method that was previously unachievable. This invention is of great industrial value for graphene applications and is expected to greatly facilitate research aimed at the utilization of graphene.

### (Example 12: Transfer of large-area graphene using a porous membrane/graphene laminated body)

In this example, a laminated body composed of a monolayer graphene film and a cellulose membrane (porous membrane) (hereinafter also referred to as a "porous membrane/graphene laminated body") was fabricated as follows.

Copper foil (65 mm × 90 mm, thickness 35 µm) was prepared as the metal catalyst substrate for chemical vapor deposition (CVD) growth of graphene, and graphene film formation by CVD was carried out in the same manner as in Example 8. Prior to forming the graphene film, the copper foil was sequentially immersed in deionized water, ethanol, and acetone, followed by ultrasonic treatment to clean the copper foil surface. Thereafter, the copper foil was transferred into a thermal CVD furnace, hydrogen was introduced, and hydrogen annealing of the copper substrate was performed under a hydrogen atmosphere at a total pressure of 700 Pa and a temperature of 1000 °C for 30 minutes. Then, methane was introduced, and CVD growth of graphene was carried out for 10 minutes at 1000 °C and a total pressure of 750 Pa, with methane and hydrogen flow rates of 2 sccm and 20 sccm, respectively.

A porous membrane was formed on the graphene film grown on the copper foil by a phase inversion method.

As the transfer support layer for graphene, a mixed film of nitrocellulose (CN) and cellulose acetate (CA) (mixed cellulose ester film (MCE)) was used. As the raw materials for the porous-membrane-forming solution, nitrocellulose (Nacalai Tesque, product No. 24728-34), cellulose acetate (Sigma-Aldrich, product No. 419028), and acetone and formamide as solvents were prepared. A porous-membrane-forming solution was prepared by dissolving cellulose acetate and nitrocellulose in a mixed solvent of acetone and formamide (1:1 by weight), so that the cellulose acetate content was 4 wt% and the nitrocellulose content was 8 wt%.

Next, the copper foil bearing a CVD-grown graphene film was fixed on the stage of an automatic linear motion coater, and the solution was uniformly spread over the graphene by means of a multi-gap applicator (width: 100 mm) under the conditions of a gap height of 100 µm and a travel speed of 15 mm/s. The graphene/copper foil laminated body coated with the solution by the applicator was immersed in a deionized water bath and left to stand at room temperature for 1 hour. The graphene/copper foil laminated body with the porous membrane formed thereon was removed from the deionized water bath, dried at room temperature, and then the graphene film formed on the opposite side of the copper foil (i.e., the side opposite to the porous membrane) was removed by oxygen plasma treatment. Subsequently, the laminated body composed of the porous membrane/graphene/copper foil was cut into 65 mm × 65 mm pieces. An etchant of 10 wt% ammonium persulfate aqueous solution was prepared in an etching bath, and the laminated body was floated with the copper foil surface in contact with the etchant. After about 4 hours, complete etching of the copper was confirmed, and while discharging the ammonium persulfate solution from the bath, deionized water was introduced to wash the porous membrane/graphene laminated body. In the laminated body thus prepared, the porous support membrane had a thickness of 10 µm or more, and it possessed sufficient strength to avoid tearing even when removed from the deionized water. After removal from deionized water, the laminated body was placed on a membrane filter having a diameter of 90 mm (ADVANTEC, product code: A045A090C) such that the porous membrane side was in contact with the surface of the membrane filter, and was then dried at room temperature. The obtained laminated body was able to stand alone in the atmosphere without undergoing irreversible deformation.

After drying the laminated body and the membrane filter, the laminated body was peeled off from the membrane, making it possible to handle the laminated body alone. To transfer this mixed cellulose ester/graphene laminated body onto a SiO₂/Si substrate (4-inch-diameter) with four locations of Cr/Au contact pads deposited on its surface, a small amount of deionized water was dropped onto the substrate, and the laminated body together with the membrane filter was placed at the desired position on the substrate with the graphene film facing the SiO₂/Si substrate. At this time, the four corners of the graphene were positioned to contact the four chromium/gold-deposited regions on the SiO₂/Si substrate. In this transfer method, which does not require floating on water, such fine adjustment of the transfer position can be easily achieved. Thereafter, deionized water was dropped onto the membrane filter to wet it, and the membrane filter was peeled from the porous membrane. The laminated body on the SiO₂/Si substrate was then dried at room temperature. After drying, the mixed cellulose ester porous membrane was dissolved by exposing the laminated body transferred to the SiO₂/Si substrate to acetone vapor. Fig. 25 shows a photograph of the monolayer graphene after dissolution of the mixed cellulose ester support membrane. The thickness of the oxide film (SiO₂ layer) of the SiO₂/Si substrate used in this example was 300 nm, which is suitable for visualizing monolayer graphene, and it was confirmed that a large graphene film of 65 mm × 65 mm was transferred. Furthermore, since the transferred graphene exhibited a uniform color and no color variation due to residues was observed, it was found that this transfer method is an excellent method with very few tears in the graphene and very little support-film residue. The sheet resistance measured by the van der Pauw method was 354 Ω/sq. Obtaining such a low sheet resistance demonstrates that the graphene/porous membrane laminated body according to the present invention enables transfer of large-area graphene with extremely few tears. Fig. 26 shows photographs of each step in the transfer process of this example.

### (Example 13: Making a transferred porous membrane/graphene laminated body transparent)

In this example, a PVDF/graphene laminated electrode was fabricated by a solution casting method using an applicator with a gap height of 100 µm, in a similar procedure as in Example 9. The PVDF porous-membrane-forming solution was prepared by dissolving PVDF (Sigma-Aldrich, product No. 347078) at 12 wt% in a mixed solvent of acetone/dimethylformamide (DMF) at a weight ratio of 1:1. The fabricated PVDF/graphene/quartz glass substrate laminated body was subjected to hydrophilic treatment by immersion in methanol, and then impregnated with either water or a water/glycerol solution, covered with a coverslip, and its transmittance was measured. The transmittance spectra are shown in Fig. 27. The transmittance at 550 nm was 39% for the laminated body impregnated with water, whereas that of the laminated body impregnated with a water/glycerol solution (4:6 by weight) was 89%. Thus, the PVDF porous membrane/graphene laminated body was rendered transparent.

### (Example 14: Evaluation of graphene transferred by the transfer method using a porous membrane/graphene laminated body through Raman mapping)

In this example, a 20 mm × 20 mm mixed cellulose ester/graphene laminated body fabricated by the solution casting method using an applicator with a gap height of 100 µm was transferred to a 20 mm × 20 mm quartz glass substrate in the same procedure as in Example 9. After removing the porous membrane by exposure to acetone vapor, Raman mapping of the graphene on the quartz glass substrate was performed using a Raman imaging microscope (alpha300, WITec) to examine the coverage of the graphene. The measurement conditions involved mapping the entire 20 mm × 20 mm quartz glass substrate at 1 mm intervals in both vertical and horizontal directions, resulting in a 20 × 20 mapping grid. An autofocus function was used for the measurements. The presence of graphene was determined by the existence of the 2D peak. Excluding the outermost grid, 19 × 19 = 361 cells were analyzed; among them, the 2D peak was absent in only one cell, and a cellulose peak was observed in one cell. Therefore, it was confirmed that more than 99% of the transferred graphene region consisted of graphene free from support-film residues and tears.

### Explanation of Reference Numerals

1 Laminated body (porous membrane/two-dimensional layered material)
1' Laminated body (porous membrane/two-dimensional layered material/metal catalyst substrate)
1" Laminated body (porous membrane/two-dimensional layered material/metal catalyst substrate; wet state)
2 Two-dimensional layered material
3 Porous membrane
3' Porous-membrane-forming solution
4 Metal catalyst substrate
5 Applicator
6 Water bath
7 Etching bath
8 Membrane
9 PMMA
10 PMMA laminated body (PMMA/graphene film/metal catalyst substrate)
10' PMMA laminated body (PMMA/graphene film)
11 Substrate

## Claims

1. A laminated body comprising a two-dimensional layered material and a porous membrane laminated on the two-dimensional layered material,
wherein the laminated body is self-supporting while maintaining a planar structure under atmospheric conditions.

2. The laminated body according to claim 1,
wherein the laminated body is in a dried state.

3. The laminated body according to claim 1,
wherein the porous membrane has pores with an average pore diameter of 20 nm or more.

4. The laminated body according to claim 1,
wherein the pore area ratio relative to the surface of the porous membrane is 20% or more.

5. The laminated body according to claim 1,
wherein the porous membrane has a film thickness of 100 nm or more.

6. The laminated body according to claim 1,
wherein the porous membrane is a membrane made of a material selected from the group consisting of nitrocellulose, cellulose acetate, polyethersulfone, polytetrafluoroethylene, polyamide, polyvinylidene fluoride, regenerated cellulose, polycarbonate, polypropylene, polyvinylidene chloride, aluminum oxide, glass fiber, quartz fiber, polymethyl methacrylate, polystyrene, polyethylene, polyethylene terephthalate, and ceramic,
or a mixed membrane made of two or more materials selected from the group.

7. The laminated body according to claim 1,
wherein the porous membrane is a membrane made of a material selected from the group consisting of nitrocellulose, cellulose acetate, polycarbonate, polyvinylidene chloride, polystyrene, and polymethyl methacrylate,
or a mixed membrane made of two or more materials selected from the group.

8. A method for transferring the laminated body according to any one of claims 1 to 7 onto a substrate,
the method comprising:
(a) adhering the two-dimensional layered material side of the laminated body to a desired position on the substrate.

9. The transfer method according to claim 8,
wherein, after step (a), the method further comprises:
(b) removing the porous membrane of the laminated body using a solvent.

10. A method for producing the laminated body according to any one of claims 1 to 7, the method comprising:
(i) forming a two-dimensional layered material on a metal catalyst substrate by a chemical vapor deposition (CVD) method;
(ii) further forming a porous membrane on the two-dimensional layered material to produce a laminated body; and
(iii) removing the metal catalyst substrate by etching.

11. The method for producing a laminated body according to claim 10,
wherein the formation of the porous membrane on the two-dimensional layered material in step (ii) is carried out by a phase inversion method.

12. The method according to claim 11,
wherein, in step (ii), a solvent for dissolving a polymer used to form the porous membrane is a mixed solvent comprising a good solvent and a poor solvent for the polymer.
